# EUROPEAN PATENT APPLICATION

(11) **EP 4 647 439 A1**
(43) Date of publication of application: **12.11.2025**
(21) Application number: 24175034.8
(22) Date of filing: 09.05.2024
(51) Int. Cl.: C07K 14/195, A61K 35/744, A61K 38/00, A61K 38/16, A61K 38/17, C07K 14/005, C07K 14/315, C07K 14/335, C07K 14/47, C12N 9/50, C12N 15/62, C12N 15/74, C12N 15/86

(54) **NON-PATHOGENIC BACTERIA FOR DELIVERY OF PTD-FUSION PROTEINS**

(71) Applicant: Alma Mater Studiorum - Università di Bologna, 40126 Bologna (IT); Jozef Stefan Institute, 1000 Ljubljana (SI)
(72) Inventor: Ciani, Elisabetta, 40126, Bologna (IT); Trazzi, Stefania, 40126, Bologna (IT); Medici, Giorgio, 40126, Bologna (IT); Candini, Giulia, 40126, Bologna (IT); Berlec, Ale, SI-1000, Ljubljana (SI); Stojanov, Spase, SI-1000, Ljubljana (SI)
(74) Representative: Currado, Luisa

(57) **Abstract**

The present invention provides the use of non-pathogenic bacteria as microbial cell factories for the live production of secretable protein transduction domain (PTD)-fusion protein, which can be used as a protein replacement therapy for a wide spectrum of monogenic pathologies, including those affecting the brain.

## Description

### FIELD OF THE INVENTION

The present invention provides the use of non-pathogenic bacteria as microbial cell factories for the live production of secretable protein transduction domain (PTD)-fusion protein, which can be used as a protein replacement therapy for a wide spectrum of monogenic pathologies, including those affecting the brain. The PTD-fusion protein, once secreted by the non-pathogenic bacteria in the host organism, by virtue of the presence of the transduction domain (PTD), can be conveyed first into the blood and subsequently to various organs, including the brain, by crossing the blood-brain barrier.

### BACKGROUND

### Rare Monogenic Diseases

A rare disease is defined by its low prevalence in the general population. Although the threshold varies slightly between different countries, a specific disease is usually considered rare when it affects fewer than 50-60 individuals out of 100,000. Many rare pathologies are of monogenic origin, i.e., due to mutations in a single gene. Although the name underlines that these diseases are rare, the reality is that mutations in 4000 different genes are at the origin of 5000-8000 pathologies that affect approximately 300 million people worldwide (Nguengang Wakap et al., 2020).

Rare genetic diseases represent at least 80% of all rare diseases and involve a broad spectrum of clinical characteristics and pathophysiological mechanisms. Surprisingly, rare monogenic diseases manifest themselves mainly in childhood and are mostly incurable. 70% of these disorders disproportionately affect the nervous system of children with devastating effects, giving rise to chronic and/or progressive and degenerative pathologies, the predominant phenotypic manifestations of which are represented by neurodevelopmental alterations (Lee et al., 2020). In particular, it is estimated that monogenic neurological disorders represent up to 40% of the workload in pediatric hospitals, with over 1% of children affected at birth. Many different forms of brain impairment are associated with these disorders including effects on brain development from birth (Fragile-X syndrome, CDKL5 deficiency disorder, some forms of Parkinson's disease or amyotrophic lateral sclerosis) and anomalies in brain functioning with early or late onset, even in the absence of structural alterations of the brain (dystonia) (doi:10.1136/bmj.m3752). The result is a wide spectrum of highly disabling symptoms such as severe intellectual disability, stereotypies, autism, epilepsy, cognitive decline, motor deficits and psychiatric disorders. Currently, the development of effective/resolving therapies represents one of the most arduous challenges in the medical and biological fields.

### Protein Replacement Therapy

In theory, for a monogenic disease, the delivery of a wild-type copy of the mutated protein to cells represents the most curative approach. However, the failure of proteins to penetrate the cell membrane prevents their use as a therapeutic tool in a variety of monogenic disorders caused by the lack of function of a single protein. Recently, protein transduction domains (PTDs) have been shown to promote the delivery of proteins into living cells and through the blood brain barrier. PTDs are small peptides that are able to ferry much larger molecules into cells independently, through classical endocytosis (Bolhassani et al., 2017). The HIV-1 transactivator of transcription (TAT) protein is the best characterized viral PTD-containing protein. It has been reported that a protein transduction domain (TAT) is able to deliver macromolecules into cells and even into the brain when fused to a given protein (Nagahara et al., 1998; Schwarze et al., 1999; Xia et al., 2001). Importantly, the TAT-PTD has not been reported to have any toxic effects or immunogenicity problems so far (Bolhassani et al., 2017). Protein therapy is often called Enzyme Replacement Therapy (ERT) when the protein features enzymatic activity. At present, ERT is a successful treatment mainly for metabolic lysosomal storage diseases such as Gaucher disease, Scheie syndrome, Maroteaux-Lamy syndrome, Morquio A Syndrome, and Sly Syndrome (Rapoport and Lorberboum-Galski, 2009). This approach consists of the trans-vascular administration of a functional therapeutic protein that is produced in a protein production system including those derived from bacteria, baculovirus/insect cells, mammalian cells, and yeast.

CDKL5 deficiency disorder (CDD) is a rare X-linked neurodevelopmental disorder (Bertani et al., 2006; Fehr et al., 2013; Guerrini and Parrini, 2012; Mari et al., 2005). It is caused by mutation(s) in the cyclin-dependent kinase-like 5 (CDKL5) gene that leads to the absence or deficiency of CDKL5, and results in a severe phenotype characterized by intellectual disability, sensorimotor impairment, visual deficits, and early-onset epilepsy. Although pharmacological treatments are used to control seizures, no cure or effective treatments are currently available to ameliorate the cognitive and behavioral symptoms of this severe disorder. In order to find a therapy for the rare monogenic disease CDKL5 deficiency disorder, inventors recently created an IgK-TATk-CDKL5 fusion construct (WO2015128746) and found that, in view of the properties of the Igk-chain leader sequence, the TATk-CDKL5 protein produced by transfected cells is secreted via constitutive secretory pathways (Trazzi et al., 2018). Importantly, due to the transduction property of the TATk peptide, the secreted CDKL5 protein is internalized by cells, maintaining its native biological activity (Trazzi et al., 2018). Notably, systemically administered TATk-CDKL5 protein passed the blood-brain-barrier, reached the CNS, and rescued various neuroanatomical and behavioral defects, including breathing pattern and visual responses (Trazzi et al., 2018). These results indicate that a protein replacement therapy, aimed at compensating for the lack of CDKL5 function, is feasible and effective, and may be a successful clinical tool for the treatment of CDKL5 deficiency disorder (CDD), and even for other rare CNS pathologies.

Despite the great potential of the protein replacement approach, one of the biggest issues affecting protein therapy is that the manufacture of drugs that leverage proteins or enzymes can be very expensive due to several technical problems such as in vitro and in vivo protein instability or solubility, and shorter half-lives. This turned out to be a problem for the recombinant TATk-CDKL5 protein; its purification in diverse platforms such as bacteria, yeast, and insect and mammalian cells was very ineffective due to the low CDKL5 protein solubility in the physiological environment, culminating in high/unsustainable production costs. Finally, this protein replacement therapy would be a lifelong treatment, entailing a daily invasive administration through intravenous, subcutaneous, or in situ injections that necessarily require large quantities of the therapeutic protein.

Ideally, therapeutic strategies able to foster the production of the protein directly inside the body of patients would overcome such hindrances.

### Lactic Acid Bacteria as a Live Delivery System

Lactic acid bacteria (LAB) have a long history of safe exploitation by humans, as they have been used for centuries in food production and preservation, and as probiotic agents to promote human health. Interestingly, some species of these Gram-positive bacteria, which are recognized as safe organisms by the US Food and Drug Administration (FDA) and by the European Food Safety Authority (EFSA), are able to survive in the gastrointestinal tract (GIT), and are capable of reaching and colonizing the intestine, where they play an important role.

Given the need to develop effective and safe strategies for the delivery of therapeutic molecules, LAB are reported to be ideal candidates as live delivery vehicles for the release of therapeutic and prophylactic molecules directly at the site of the oral, nasal, and genital mucosae and are a realistic option for the treatment of human diseases (Cano-Garrido et al., 2015; Wang et al., 2016). The safety status of LAB, the ability of some strains to survive passage through the GIT (Vesa et al., 2000), and the capacity of some species to remain viable in the GIT for a period of time render food-grade LAB ideal vehicles with which to deliver and even produce therapeutic molecules in situ at the GIT mucosa (Wang et al., 2016). The absence of lipopolysaccharides (LPSs) in their cell walls is a further advantage, allowing them to be administered orally without the risk of endotoxic shock (Szatraj et al., 2017). Therefore, the oral administration of therapeutic molecules via live recombinant LAB may be a suitable alternative to invasive administration methods, for example, parenteral or subcutaneous injection, thus avoiding their potential side effects. Furthermore, it circumvents the degradation of orally administered unprotected molecules in the digestive tract and ensures the production of the therapeutic protein at the GIT mucosa (Wang et al., 2016).

Another advantage of the in vivo synthesis of the therapeutic molecule is a significant decrease in the production cost of treatment agents since, as they are live organisms, LAB would be able to autonomously multiply, produce, and deliver the protein of interest, thus avoiding pharmaceutical production and purification of active agents, and overcoming degradation problems which are particularly severe in the gastrointestinal tract.

In recent decades, much effort has gone into the genetic manipulation of LAB with the aim of producing recombinant therapeutic proteins (Cano-Garrido et al., 2015; Wang et al., 2016) that can be engineered to be secreted into the extracellular environment. Recently, the inventors have used principles of synthetic biology to engineer LAB, *Lactococcus lactis,* to secrete and, optionally, anchor onto its surface small protein binders of pro-inflammatory signaling molecules, cytokines, and chemokines. The platform for recombinant protein binder secretion using Usp45 secretion signal and cAcmA surface anchor (patent US 8754198 B2) was used to target TNF, IL-6, IL-8, IL-17, and other cytokines and chemokines, all important players involved in the pathology of inflammatory bowel disease (Skrlec et al., 2017; Zahirovic et al., 2022). The approach was tested in the delivery of TNF-binding affibody to the gastrointestinal mucosa of mice, and significant interference with TNF signaling was observed, mimicking that of parenterally administered monoclonal antibody (Berlec et al., 2017).

The development of effective therapies for rare monogenic diseases represents a major social challenge. Given the need to develop effective and safe strategies for the administration of therapeutic molecules for the treatment of rare diseases, in particular for monogenic neurological disorders, probiotic agents, such as LAB, recognized as safe organisms for human nutrition, are becoming a realistic option for the oral administration of therapeutic molecules. Although, in recent decades, the use of LAB for the *in situ* production of therapeutic molecules within the body has been widely developed, and some of these approaches have reached a clinical development (Bron and Kleerebezem, 2018; Charbonneau et al., 2020; Song et al., 2017), all of them are focused at GIT disorders. The protein produced by LAB in these cases acts directly in situ on the intestinal mucosa and is not required to reach organs/tissues that are distant from the site of production by LAB.

### SUMMARY OF THE INVENTION

The present invention provides a novel approach, based on the use of non-pathogenic bacteria as microbial cell factories for the live production of secretable PTD-fusion protein, which lays the foundations for the development of a protein replacement therapy that may be feasible for a wide spectrum of monogenic pathologies, including those affecting the brain. In an embodiment the invention provides genetically modified lactic acid bacteria expressing TATk-fusion protein encoded by a gene construct coding for a polypeptide comprising a secretion signal peptide and a TATk-fusion protein under the control of a suitable promoter.

It is therefore an object of the invention a non-pathogenic bacterium genetically modified to express a Protein Fusion Domain (PTD)-fusion protein wherein said non-pathogenic bacterium comprises a gene construct coding for a secretion signal peptide and for a PTD-fusion protein, said gene construct being under the control of a suitable promoter.

Preferably the encoded PTD-fusion protein is a TATk-fusion protein.

In a preferred embodiment the encoded TATk-fusion protein comprises:
- a secretion signal peptide of USP45 with at least 70%, at least 80% or at least 90% sequence identity to SEQ ID No. 27;
- a TATk polypeptide sequence with at least 70%, at least 80% or at least 90% sequence identity to SEQ ID No. 26;
- a protein suitable for protein replacement therapy, preferably said protein being selected from a CDKL5 polypeptide sequence, alpha1-Antitrypsin, β-Glucocerebrosidase, Adenosine Deaminase, Alpha-Galactosidase A, Acid alpha-Glucosidase, α-L-Iduronidase, Iduronate-2-Sulfatase, N-Acetylgalactosamine-6 Sulfatase, N-Acetylgalactosamine-4 Sulfatase, Lysosomal Acid Lipase, MECP2 (methyl-CpG binding protein 2), KDM5C (lysine demethylase 5C), CASK (Calcium/calmodulin-dependent serine protein kinase), SERPINA1 (serine protease inhibitor a1-antitrypsin (AAT) protein), DNAI1 (Dynein Axonemal Intermediate Chain 1), CYBB (cytochrome b-245, beta chain), XPA (DNA repair protein complementing XP-A cells), and
wherein the TATk is operatively coupled to the protein suitable for protein replacement therapy. It is a further object of the invention the non-pathogenic bacterium as defined above wherein the encoded PTD-fusion protein further comprises a reporter protein polypeptide wherein the reporter protein polypeptide is operatively coupled to the N-terminus or to the C-terminus of the PTD-fusion protein.

It is a further object of the invention the non-pathogenic bacterium as defined above wherein the encoded TATk fusion protein has or comprises a polypeptide sequence with at least 70%, at least 80% or at least 90% sequence identity to SEQ ID No. 23 or to SEQ ID No. 25.

It is a further object of the invention the non-pathogenic bacterium as defined above being a lactic acid bacterium (LAB), preferably said LAB is *Lactococcus lactis.*

It is a further object of the invention the non-pathogenic bacterium as defined above for use as a medicament, preferably for use as a delivery vector for protein replacement therapy in the treatment of genetic disease, preferably in the treatment of rare monogenic diseases.

In a preferred embodiment the rare monogenic diseases are neurological diseases preferably selected from Fragile-X syndrome, CDKL5 deficiency disorder, Rett syndrome, Lysosomal storage diseases, Claes-Jensen type (MRXSCJ); CASK-related disorders; α1-antitrypsin deficiency; Kartagener syndrome; Chronic Granulomatous Disease; Xeroderma Pigmentosum, Gaucher disease, Pompe disease, ADA deficiency.

It is a further object of the invention a pharmaceutical composition comprising a therapeutically effective amount of the non-pathogenic bacterium as defined above and a pharmaceutically acceptable carrier.

Preferably said pharmaceutical composition is for use in the treatment of genetic disease, preferably in the treatment of rare monogenic diseases, preferably wherein the rare monogenic diseases are neurological diseases preferably selected from Fragile-X syndrome, CDKL5 deficiency disorder, Rett syndrome, Lysosomal storage diseases, Claes-Jensen type (MRXSCJ); CASK-related disorders; α1-antitrypsin deficiency; Kartagener syndrome; Chronic Granulomatous Disease; Xeroderma Pigmentosum, Gaucher disease, Pompe disease, ADA deficiency.

It is a further object of the invention a method for producing the non-pathogenic bacterium as defined above comprising introducing into said lactic acid bacterium a gene construct coding for a secretion signal peptide and for a TATk-fusion protein, said gene construct being under the control of a suitable promoter, preferably wherein the genetic construct comprises a sequence with at least 70%, at least 80% or at least 90% sequence identity to any one of SEQ ID No 16, SEQ ID No. 18, SEQ ID No 22, SEQ ID NO 24.

Preferably said gene construct is in a plasmid vector capable of replicating in said lactic acid bacterium or said gene construct is integrated into the genome of said lactic acid bacterium.

It is a further object of the invention a food product comprising the non-pathogenic bacterium as defined above, preferably said food product being selected from the group consisting of milk, yogurt, curd, cheese, fermented milks, milk based fermented products and milk-based powders. It is a further object of the invention the use of the non-pathogenic bacterium as defied above as base ingredient for the preparation of nutraceutical products.

The invention will be discussed by reference to the following figures.

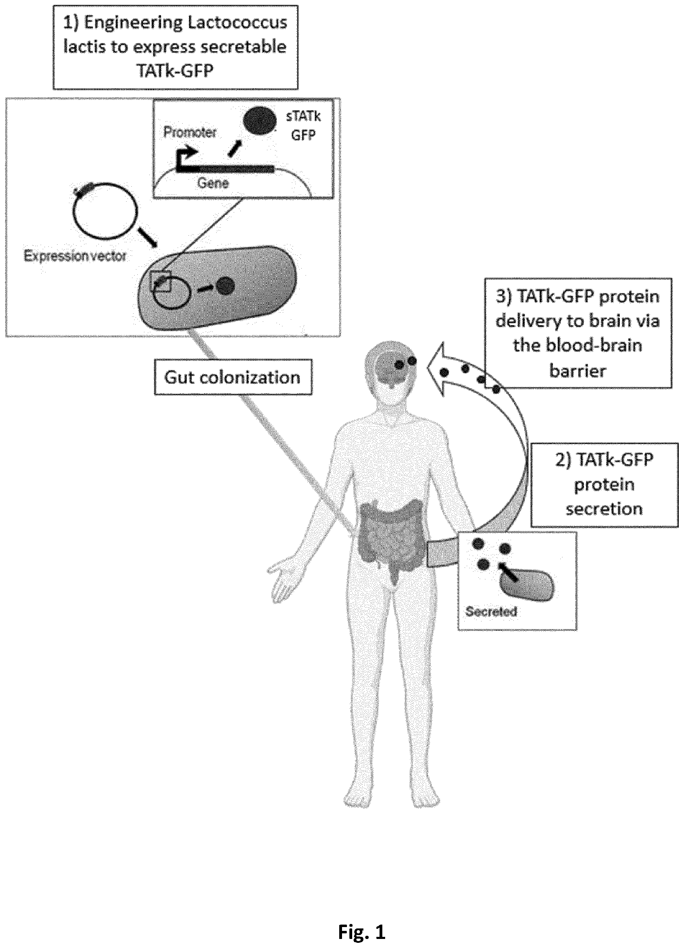

**Figure 1****.** Diagram of the in vivo delivery of TATk-GFP protein by *Lactococcus lactis.*

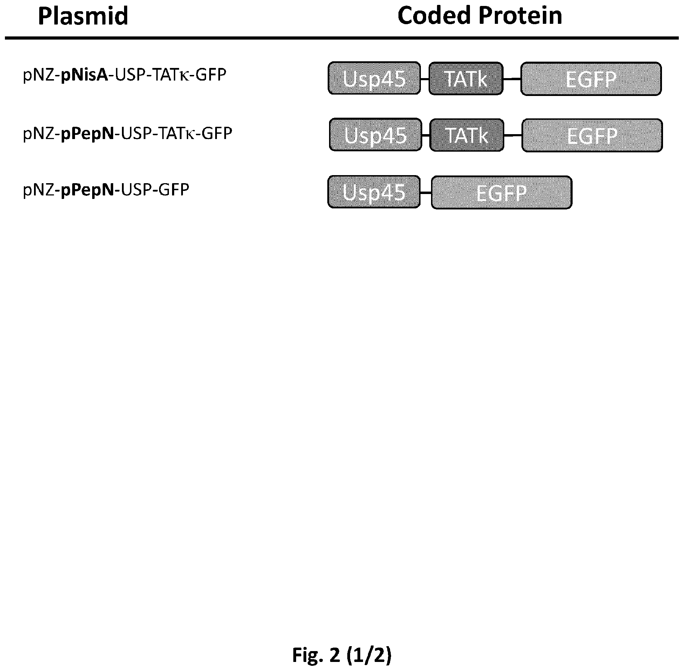

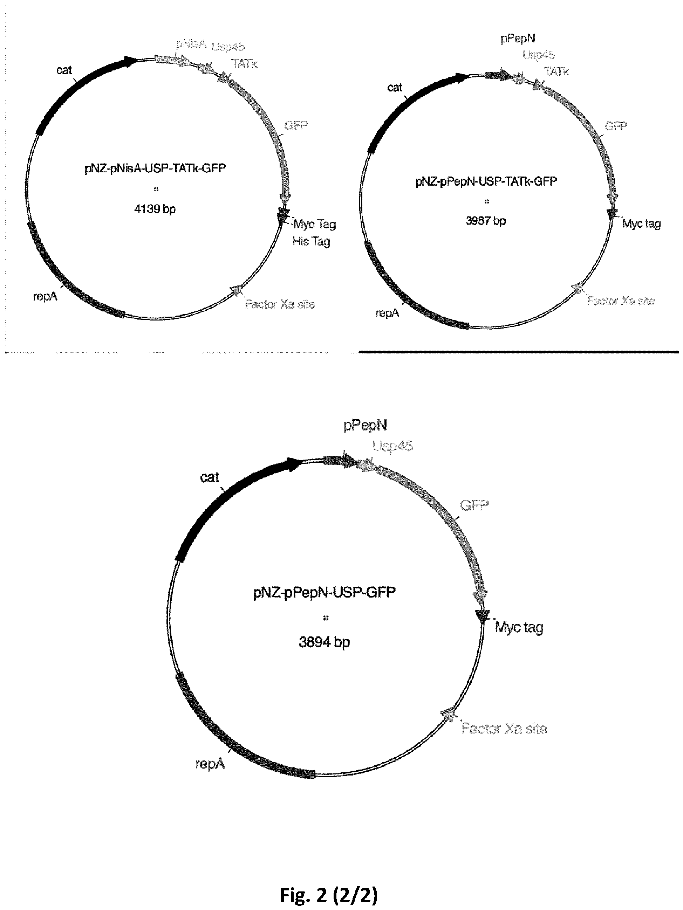

**Figure 2****.** Schematic representation of constructs used to engineer the *L. lactis* to express the TATk-GFP and GFP proteins and plasmid schemes. Image shows construct names, displaying plasmid name (pNZ), type of promoter (inducible - pNisA or constitutive - pPepN) and coded fusion proteins. Plasmid Fusion proteins are composed of peptides represented as colored rounded squares. All the constructs express secretable proteins bearing the signal peptide for secretion (Usp45); two of these carry a protein transduction domain (TAT_{K} peptide).

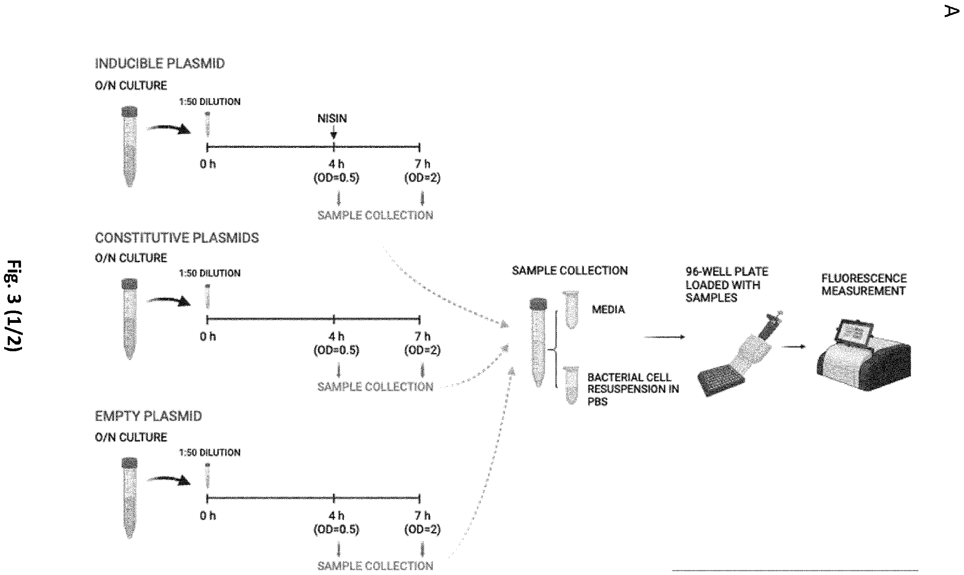

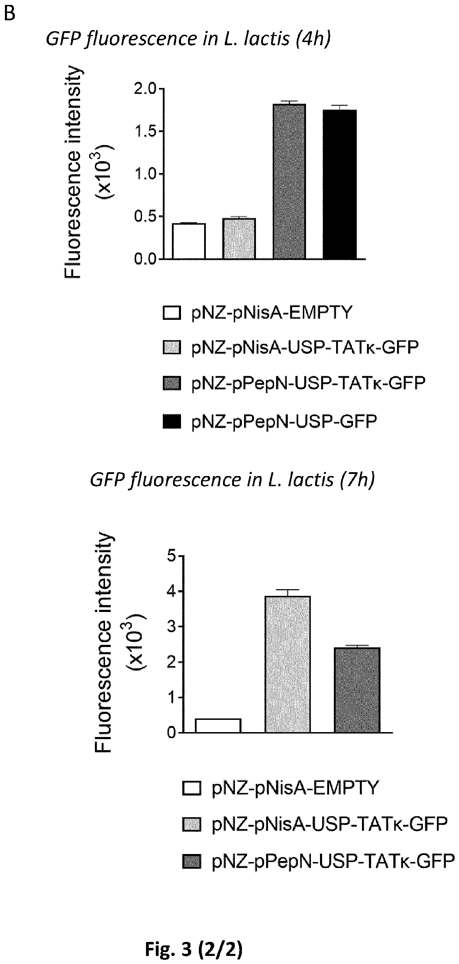

**Figure 3****.** TATk-GFP expression and secretion. **A:** Schematic representation of the protocols used for GFP fluorescence measurement from *L. lactis* cultures harboring inducible, constitutive, and empty plasmids. Abbreviation: O/N, overnight. **B:** Fluorescence intensity in *L. lactis* harboring the different TATk-GFP and GFP constructs or an pNZ-empty plasmid as a negative control. Samples were collected after 4 (4h) or 7 (7h) hours of growth with or without nisin induction as in A.

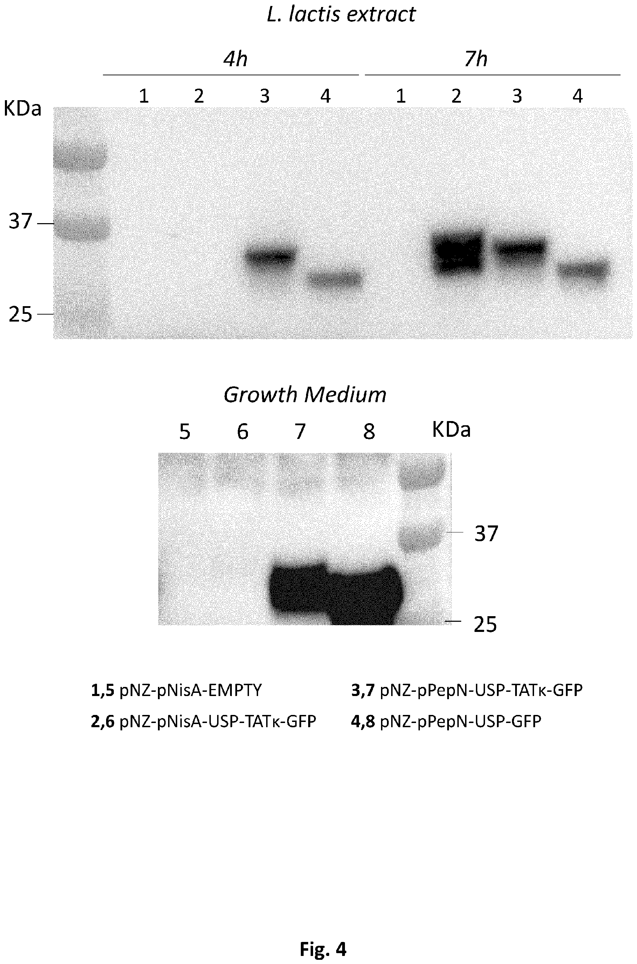

**Figure 4****.** Western blot analysis of TATk-GFP and GFP levels in protein extracts from bacterial cell resuspension of *L. lactis* harboring the empty plasmid (line 1) or the pNZ-pNisA-USP-TATk-GFP, pNZ-pPepN-USP-TATk-GFP, or pNZ-pPepN-USP-GFP plasmid (lines 2-4) after 2 or 7 hours of growth. After 7 hours of growth, with or without induction with nisin as in Fig. 3A, the medium of *L. lactis* harboring the empty plasmid (line 5) or the pNZ-pNisA-USP-TATk-GFP, pNZ-pPepN-USP-TATk-GFP, or pNZ-pPepN-USP-GFP plasmid (lines 6-8) was collected and concentrated 100x. Anti-GFP antibody was used to detect TATk-GFP and GFP protein levels.

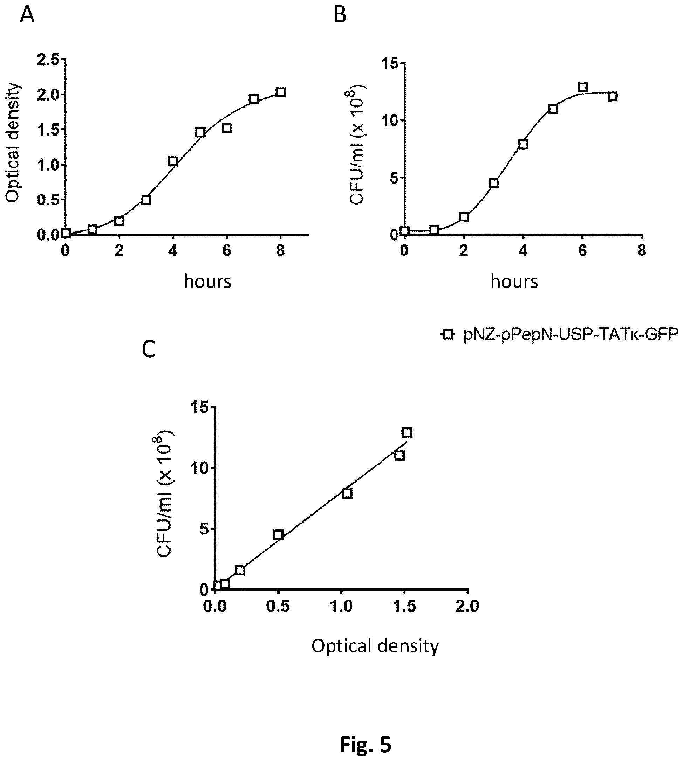

**Figure 5****.** Effect of TATk-GFP expression on *L. lactis* growth and viability. Overnight cultures of *L. lactis* harboring the pNZ-pPepN-USP-TATk-GFP plasmid were grown in GM17 medium for 8 hours. Bacterial cell density/viability was measured every hour. **A:** Optical density (OD₆₀₀) over incubation time (h) plot for the growth of *L. lactis* at 30°C. **B:** Numbers of *L. lactis* with time, expressed as colony forming units (CFUs)/ml, at time intervals of 1 hour. *L. lactis* cultures were plated on GM17 plates and grown at 30°C for 48 hours. C: Plot of the observed OD₆₀₀ against the *L. lactis* number (CFU/ml).

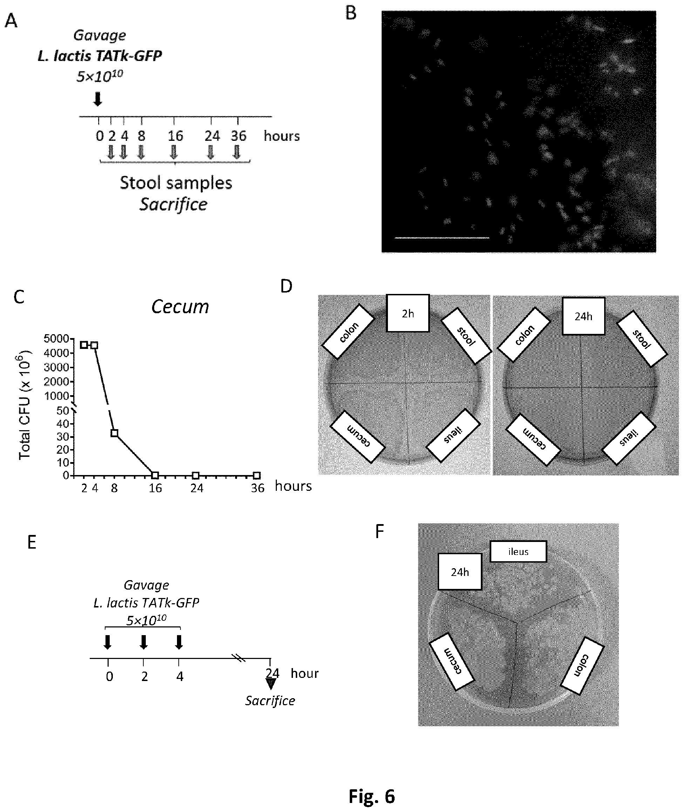

**Figure 6****.** Efficacy of intestinal colonization of recombinant *L. lactis* expressing the TATk-GFP protein. **A:** Schematic representation of the experimental plan. Mice were administered (orally) with 5 × 10¹⁰ cells of recombinant *L. lactis* and sacrificed at different time points (indicated by arrows). **B:** Representative image of a GFP-stained section, showing *L. lactis* expressing the TATk-GFP protein in a portion of the cecum. Scale bar = 15 µm. **C:** Quantification of numbers of recombinant *L. lactis* in the cecum at different time points after oral gavage, expressed as colony forming units (CFUs/ml). **D:** General appearance of the bacterial colonies, from the intestinal content (ileus, cecum, and colon) and stool samples collected 2 or 24 hours after the administration of recombinant *L. lactis,* and grown for 48 hours on selective GM17 plates supplemented with chloramphenicol. Please note the absence of *L. lactis* in the ileus 24 h after gavage. **E:** Schematic representation of the experimental plan. Mice were administered (orally) with 5 × 10¹⁰ cells of recombinant *L. lactis* 3 times, once every 2 hours (black arrows), and sacrificed 24 hours after the first administration. **F:** General appearance of the bacterial colonies from the intestinal content (ileus, cecum, and colon), collected 24 hours after the first administration of recombinant *L. lactis* according to the administration schedule in E.

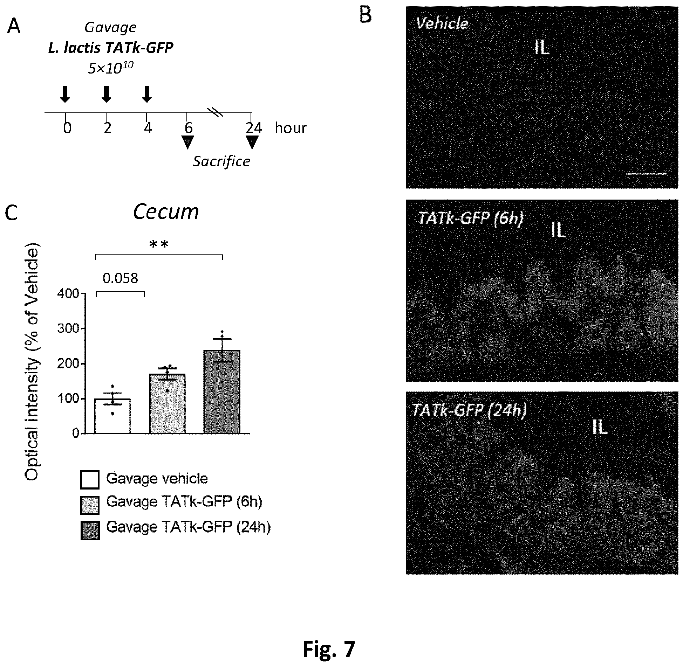

**Figure 7****.** In vivo secretable TATk-GFP protein biodistribution. **A:** Mice were administered (orally) with 5 × 10¹⁰ cells of recombinant *L. lactis* harboring the pNZ-pPepN-USP-TATk-GFP plasmid 3 times, once every 2 hours (black arrows), and sacrificed 2 hours (6h) or 20 hours (24h) after the last administration. **B:** Representative images of GFP-immunostained cecum sections of a mouse administered with recombinant *L. lactis* or vehicle (PBS), treated as described in A. Scale bar = 70 µm. **C:** Quantification of the mean intensity of GFP immunoreactivity per area in the cecum of mice administered with recombinant *L. lactis* or vehicle as described in A (vehicle, n = 4; *L. lactis* TATk-GFP, n = 4). Values are represented as means ± SEM. ** p<0.01 (Fisher's LDS test after one-way ANOVA). Abbreviation: IL = intestinal lumen.

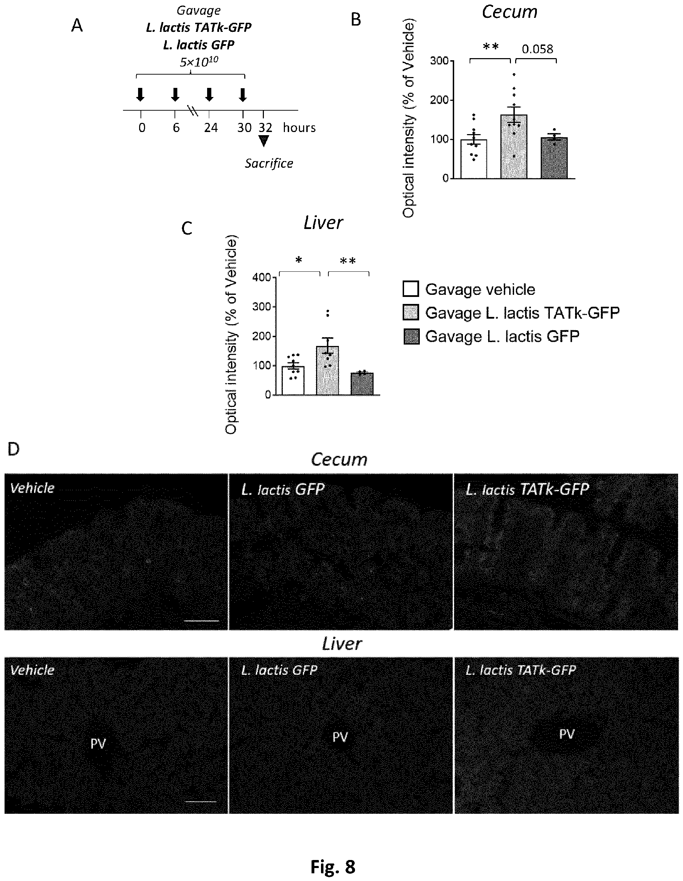

**Figure 8****.** In vivo secretable TATk-GFP and GFP protein biodistribution. **A:** Schematic representation of the experimental plan. Mice were administered with 5 × 10¹⁰ cells of recombinant *L. lactis* harboring the pNZ-pPepN-USP-TATk-GFP plasmid, the pNZ-pPepN-USP-GFP plasmid, or vehicle (PBS) as a negative control, twice per day, with a 6-hour interval, for 2 consecutive days (black arrows). Mice were sacrificed 2 hours after the last administration. **B,C:** Quantification of the mean intensity of GFP immunoreactivity per area in the cecum and liver of mice administered with recombinant *L. lactis* or vehicle as described in A. **D:** Representative images of GFP-stained cecum and liver sections of a mouse administered with recombinant *L. lactis* or vehicle as described in A. Scale bar = 70 µm. Values are represented as means ± SEM. * p<0.05, ** p<0.01 (Fisher's LDS test after one-way ANOVA). Abbreviation: PV = portal vain.

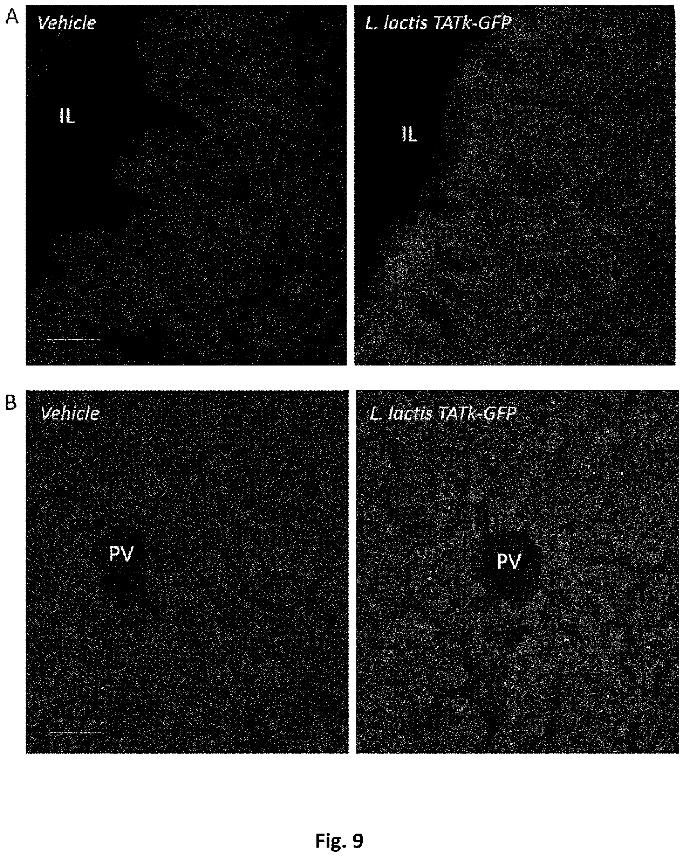

**Figure 9****.** Mice were administered with 5 × 10¹⁰ cells of recombinant *L. lactis* harboring the pNZ-pPepN-USP-TATk-GFP plasmid or vehicle (PBS, as a negative control) twice per day, with a 6-hour interval, for 4 consecutive days. Mice were sacrificed 2 hours after the last administration. **A:** Representative images of GFP-stained cecum sections of a mouse administered with recombinant *L. lactis* or vehicle for 4 consecutive days. Scale bar = 60 µm. **B:** Representative images of GFP-stained liver sections of a mouse administered with recombinant *L. lactis* or vehicle as in A. Please note the higher TATk-GFP immunoreactivity in cells close to the border delimiting the portal veins (PV), suggesting diffusion of TATk-GFP protein from the intestinal-derived bloodstream. Scale bar = 55 µm.

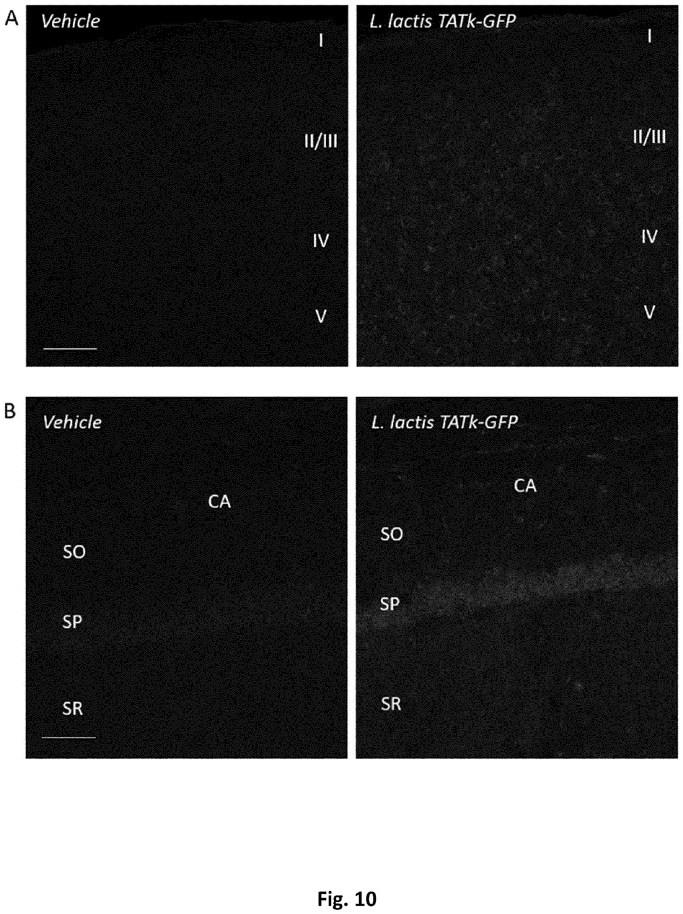

**Figure 10****.** Mice were administered with 5 × 10¹⁰ cells of recombinant *L*. *lactis* harboring the pNZ-pPepN-USP-TATk-GFP plasmid or vehicle (PBS, as a negative control) twice per day, with a 6-hour interval, for 4 consecutive days. Mice were sacrificed 2 hours after the last administration. **A,B:** Representative images of GFP-stained cortex **(A)** and hippocampus **(B)** sections of a mouse administered with recombinant *L. lactis* or vehicle. Abbreviations: CA, cornu ammonis; SO, stratum oriens; SP, stratum pyramidale; SR, stratum radiatum. Roman numerals indicate cortical layers. Scale bar = 60 µm.

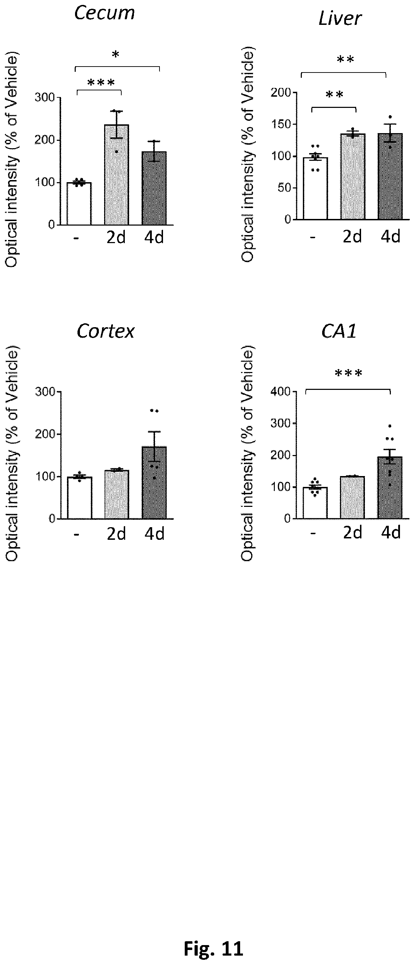

**Figure 11****.** Mice were administered with 5 × 10¹⁰ cells of recombinant *L. lactis* harboring the pNZ-pPepN-USP-TATk-GFP plasmid or vehicle (PBS, as a negative control) twice per day, with a 6-hour interval, for 2 (2d) or 4 (4d) consecutive days. Mice were sacrificed 2 hours after the last administration. Quantification of the mean intensity of GFP immunoreactivity per area in the cecum, liver, cortex, and CA1 field of the hippocampus of mice administered with recombinant TATk-GFP *L. lactis.*

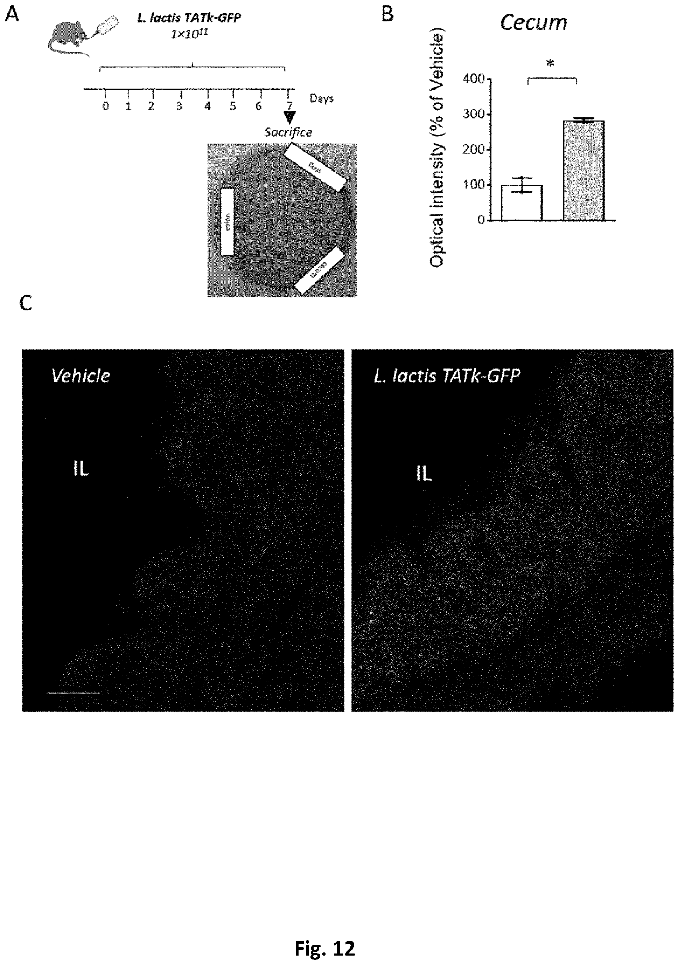

**Figure 12****.** In vivo delivery of *L. Lactis* in drinking water. **A:** Schematic representation of the experimental plan. Mice were administered for 7 days with 1 × 10¹¹ cells of recombinant *L. lactis* harboring the pNZ-pPepN-USP-TATk-GFP plasmid or vehicle resuspended in drinking water. Mice were sacrificed on day 7 and intestinal contents were collected and plated on GM17 plates supplemented with chloramphenicol. **B:** Quantification of the mean intensity of GFP immunoreactivity per area in the cecum of mice administered with recombinant *L. lactis* or vehicle as described in A. **C:** Representative images of GFP-stained cecum sections of a mouse administered with recombinant *L. lactis* or vehicle as described in A. Values are represented as means ± SEM. * p<0.05 (two-tailed Student's t-test). Abbreviation: IL = intestinal lumen. Scale bar = 60 µm

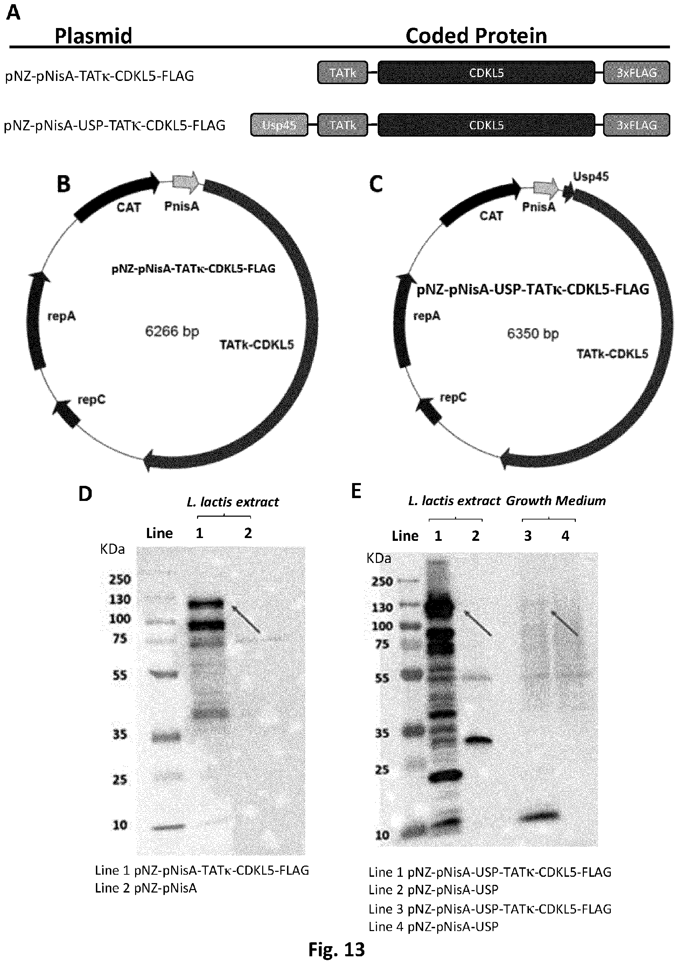

**Figure 13****.** Expression and secretion of TATκ-CDKL5 in *L. lactis.* **A,C:** Plasmid scheme for TATκ-CDKL5 cytoplasmic expression **(A,B)** and secretion **(A,C).** Western blot analysis of TATκ-CDKL5 expression. **D:** Intracellular TATκ-CDKL5 expression driven from pNZ-pNisA-TATκ-CDKL5-FLAG and pNZ-pNisA plasmids (anti-CDKL5 antibody). **E:** TATκ-CDKL5 expression driven from pNZ-pNisA-USP-TATκ-CDKL5-FLAG and pNZ-pNisA-USP plasmids and its secretion into the growth medium (cell lysate and concentrated medium, anti-Flag antibody).

### DESCRIPTION OF THE INVENTION

The present invention provides a pioneering approach, based on the use of non-pathogenic bacteria as microbial cell factories for the live production of secretable PTD-fusion protein, which lays the foundations for the development of a protein replacement therapy that may be feasible for a wide spectrum of monogenic pathologies, including those affecting the brain. In a preferred embodiment the invention provides genetically modified non-pathogenic bacteria, preferably lactic acid bacteria, expressing TATk-fusion protein encoded by a gene construct coding for a polypeptide comprising a secretion signal peptide and a TATk-fusion protein under the control of a suitable promoter.

The TATk-fusion protein, once secreted by the bacteria in the host organism, by virtue of the presence of the TATk peptide, can be conveyed first into the blood and subsequently to various organs, including the brain, by crossing the blood-brain barrier (Fig. 1).

As used herein, "non-pathogenic bacteria" refer to bacteria that are not capable of causing disease or harmful responses in a host. In some embodiments, non-pathogenic bacteria are Gram-negative bacteria. In some embodiments, non-pathogenic bacteria are Gram-positive bacteria. In some embodiments, non-pathogenic bacteria do not contain lipopolysaccharides (LPS). In some embodiments, non-pathogenic bacteria are commensal bacteria. Examples of non-pathogenic bacteria include, but are not limited to certain strains belonging to the genus Bacillus, Bacteroides, Bifidobacterium, Brevibacteria, Clostridium, Enterococcus, Escherichia coli, Lactobacillus, Lactococcus, Saccharomyces, and Staphylococcus, e.g., Bacillus coagulans, Bacillus subtilis, Bacteroides fragilis, Bacteroides subtilis, Bacteroides thetaiotaomicron, Bifidobacterium bifidum, Bifidobacterium infantis, Bifidobacterium lactis, Bifidobacterium longum, Clostridium butyricum, Enterococcus faecium, Escherichia coli, Escherichia coli Nissle, Lactobacillus acidophilus, Lactobacillus bulgaricus, Lactobacillus casei, Lactobacillus johnsonii, Lactobacillus paracasei, Lactobacillus plantarum, Lactobacillus reuteri, Lactobacillus rhamnosus, Lactococcus lactis and Saccharomyces boulardii (Sonnenborn et al., 2009; Dinleyici et al., 2014; U.S. Patent No. 6,835,376; U.S. Patent No. 6,203,797; U.S. Patent No. 5,589,168; U.S. Patent No. 7,731,976). Non-pathogenic bacteria also include commensal bacteria, which are present in the indigenous microbiota of the gut. In one embodiment, the disclosure further includes non-pathogenic Saccharomyces, such as Saccharomyces boulardii. Naturally pathogenic bacteria may be genetically engineered to reduce or eliminate pathogenicity.

In a preferred embodiment the invention relates to lactic acid bacteria (LAB) as the non-pathogenic bacteria. Lactic acid bacteria are a group of phylogenetically related microorganisms, gram-positive, either rod-shaped (bacilli) or spherical (cocci) bacteria that share common metabolic and physiological characteristics. These bacteria, usually found in decomposing plants and milk products, produce lactic acid as the major metabolic end product of carbohydrate fermentation, giving them the common name lactic acid bacteria (LAB). LAB includes: Carnobacterium, Enterococcus, Lactobacillus, Lactococcus, Leuconostoc, Oenococcus, Pediococcus, Streptococcus, Tetragenococcus, Vagococcus and Weisela. According to their physiological characteristics, genus Bifidobacterium spp. is usually classified as lactic acid bacterium because it occupies the same environmental niches, even though it is phylogenetically more distant. A preferred LAB according to the invention is Lactococcus species. In one preferred embodiment said LAB is Lactococcus lactis strain.

As used herein, "DNA molecule" includes nucleic acids/polynucleotides that are made of DNA. As used herein, "gene" refers to a specific sequence of nucleotides in DNA that is located usually on a chromosome and that is the functional unit of inheritance controlling the transmission and expression of one or more traits by specifying the structure of a particular polypeptide and especially a protein or controlling the function of other genetic material. Genes are made up of sequences of DNA and are arranged, one after another, at specific locations on chromosomes in the nucleus of cells. They contain information for making specific proteins that lead to the expression of a particular physical characteristic or trait, such as hair color or eye color, or to a particular function in a cell.

As used herein, the term "recombinant" generally refers to a non-naturally occurring nucleic acid, nucleic acid construct, or polypeptide. Such non-naturally occurring nucleic acids may include natural nucleic acids that have been modified, for example that have deletions, substitutions, inversions, insertions, etc., and/or combinations of nucleic acid sequences of different origin that are joined using molecular biology technologies (e.g., a nucleic acid sequences encoding a fusion protein e.g., a protein or polypeptide formed from the combination of two different proteins or protein fragments), the combination of a nucleic acid encoding a polypeptide to a promoter sequence, where the coding sequence and promoter sequence are from different sources or otherwise do not typically occur together naturally (e.g., a nucleic acid and a constitutive promoter, etc.). Recombinant also refers to the polypeptide encoded by the recombinant nucleic acid. Non-naturally occurring nucleic acids or polypeptides include nucleic acids and polypeptides modified by man.

A variety of viral and cellular proteins possess basic polypeptide sequences that mediate translocation across cellular membranes. The capacity to translocate across cellular membranes has become an important tool for the delivery of high molecular weight polypeptides across membranes. The phrase "protein transduction domain" (PTD) is usually used to refer to short peptides (< 30 amino acids) that can traverse the plasma membrane of many, if not all, mammalian cells. These peptides could have therapeutic potential if used in combination with other proteins that are difficult to deliver to intracellular targets. The most frequent experimental uses of PTDs are TAT, Antennapedia (Antp), and other poly-arginine peptides. Thus far, TAT has been the best characterized of the PTDs, and has been used to successfully deliver small cargoes, such as short peptides and oligonucleotides, to intercellular targets. HIV-TAT (HIV Transactivator of Transcription) is an 86-amino acid protein (Virus Res. 2019 October 15; 272: 197727, doi: 10.1016/j.virusres.2019.197727) involved in the replication of human immunodeficiency virus type 1 (HIV-1), and many studies have shown that TAT is able to translocate through the plasma membrane and reach the nucleus in order to activate transcription of the viral genome.

As used herein, "fusion protein" refers to a protein formed from the combination of at least two different proteins or protein fragments. A fusion protein is encoded by a recombinant DNA molecule. As such, a "TATk fusion protein" refers to a recombinant protein comprising the TATk polypeptide sequence YARKAARQARA (SEQ ID No. 26) or variant thereof operatively linked to other polypeptide sequences. Preferably a TATk fusion protein refers to a recombinant protein having the TATk or variant thereof operatively linked to a human CDKL5 polypeptide or variant thereof, wherein the CDKL5 polypeptide has about 50% to 100% sequence identity to SEQ ID No. 29 or SEQ ID No 30.

As used herein a "protein suitable for protein replacement therapy" is a protein used to replace a missing or deficient enzyme in a person with an inherited enzyme deficiency syndrome. This protein replacement therapy is called also Enzyme Replacement Therapy (ERT) and a list of representative proteins suitable for replacement therapy is provided below (https://www.ncbi.nlm.nih.gov/books/NBK548796/):

| **Enzyme** | **Disease** |
|---|---|
| Alpha1-Antitrypsin | A1AT Deficiency |
| β-Glucocerebrosidase | Gaucher |
| Adenosine Deaminase | ADA Deficiency |
| Alpha-Galactosidase A | Fabry |
| Acid alpha-Glucosidase | Pompe |
| α-L-Iduronidase | Hurler, MPS I |
| Iduronate-2-Sulfatase | Hunter, MPS II |
| N-Acetylgalactosamine-6 Sulfatase | Morquio Snydrome A, MPS IVA |
| N-Acetylgalactosamine-4 Sulfatase | Maroteaux-Lamy, MPS VI |
| Lysosomal Acid Lipase | Wolman, LAL Deficiency |

Further proteins suitable for protein replacement therapy are: MECP2 (methyl-CpG binding protein 2; UniProtKB: P51608), KDM5C (lysine demethylase 5C; UniProtKB: P41229), CASK (Calcium/calmodulin-dependent serine protein kinase; UniProtKB: 014936), SERPINA1 (serine protease inhibitor a1-antitrypsin (AAT) protein; UniProtKB: P01009), DNAI1 (Dynein Axonemal Intermediate Chain 1; UniProtKB: Q9UI46), CYBB (cytochrome b-245, beta chain; UniProtKB: P04839), XPA (DNA repair protein complementing XP-A cells; UniProtKB: P23025).

As used herein, "CDKL5 deficiency" refers to any deficiency in the biological function of the protein. The deficiency can result from any DNA mutation in the DNA coding for the protein or a DNA related regulatory region or any change in the function of the protein due to any changes in epigenetic DNA modification, including but not limited to DNA methylation or histone modification, any change in the secondary, tertiary, or quaternary structure of the CDKL5 protein, or any change in the ability of the CDKL5 protein to carry out its biological function as compared to a wild-type or normal subject.

As used herein, "Rett syndrome variant," "variant of Rett syndrome," and the like refers to an atypical form of Rett syndrome with similar clinical signs to Rett syndrome but an unknown etiology.

As used herein, "CDKL5 mutation" refers to any change in the nucleotide sequence of the coding region of the CDKL5 protein.

As used herein, "variant" refers to a polypeptide that differs from a reference polypeptide, but retains essential properties. A typical variant of a polypeptide differs in amino acid sequence from another reference polypeptide. Generally, differences are limited so that the sequences of the reference polypeptide and the variant are closely similar overall and, in many regions, identical. A variant and reference polypeptide may differ in amino acid sequence by one or more modifications (e.g., substitutions, additions, and/or deletions). A substituted or inserted amino acid residue may or may not be one encoded by the genetic code. A variant of a polypeptide may be naturally occurring such as an allelic variant, or it may be a variant that is not known to occur naturally. "Variant" includes functional and structural variants.

As used herein, "identity," is a relationship between two or more polypeptide sequences, as determined by comparing the sequences. In the art, "identity" also refers to the degree of sequence relatedness between polypeptide as determined by the match between strings of such sequences. "Identity" can be readily calculated by known methods, including, but not limited to, those described in (Computational Molecular Biology, Lesk, A. M., Ed., Oxford University Press, New York, 1988; Biocomputing: Informatics and Genome Projects, Smith, D. W., Ed., Academic Press, New York, 1993; Computer Analysis of Sequence Data, Part I, Griffin, A. M., and Griffin, H. G., Eds., Humana Press, New Jersey, 1994; Sequence Analysis in Molecular Biology, von Heinje, G., Academic Press, 1987; and Sequence Analysis Primer, Gribskov, M. and Devereux, J., Eds., M Stockton Press, New York, 1991 ; and Carillo, H., and Lipman, D., SIAM J. Applied Math. 1988, 48: 1073). Preferred methods to determine identity are designed to give the largest match between the sequences tested. Methods to determine identity are codified in publicly available computer programs. The percent identity between two sequences can be determined by using analysis software (e.g., Sequence Analysis Software Package of the Genetics Computer Group, Madison Wis.) that incorporates the Needelman and Wunsch, (J. Mol. Biol., 1970, 48: 443-453,) algorithm (e.g., NBLAST, and XBLAST). The default parameters are used to determine the identity for the polypeptides of the present disclosure.

As used herein, "plasmid" as used herein refers to a non-chromosomal double-stranded DNA sequence including an intact "replicon" such that the plasmid is replicated in a host cell.

As used herein, the term "vector" is used in reference to a vehicle used to introduce an exogenous nucleic acid sequence into a cell. A vector may include a DNA molecule, linear or circular (e.g. plasmids), which includes a segment encoding a polypeptide of interest operatively linked to additional segments that provide for its transcription and translation upon introduction into a host cell or host cell organelles. Such additional segments may include promoter and terminator sequences, and may also include one or more origins of replication, one or more selectable markers, an enhancer, a polyadenylation signal, etc. Expression vectors are generally derived from bacterial or yeast genomic or plasmid DNA, or viral DNA, or may contain elements of both.

As used herein, "operatively linked" indicates that the regulatory sequences useful for expression of the coding sequences of a nucleic acid are placed in the nucleic acid molecule in the appropriate positions relative to the coding sequence so as to effect expression of the coding sequence. This same definition is sometimes applied to the arrangement of coding sequences and transcription control elements (e.g. promoters, enhancers, and termination elements), and/or selectable markers in an expression vector.

As used herein, "wild-type" is the typical form of an organism, variety, strain, gene, protein, or characteristic as it occurs in nature, as distinguished from mutant forms that may result from selective breeding or transformation with a transgene.

As used herein, "cDNA" refers to a DNA sequence that is complementary to a RNA transcript in a cell. It is a man-made molecule. Typically, cDNA is made in vitro by an enzyme called reverse-transcriptase using RNA transcripts as templates.

A genetically modified non-pathogenic bacterium, such as LAB, expressing TATk, may be prepared by introduction of gene construct to the mentioned bacterium. The gene construct comprises secretion signal peptide coding sequence and TATk-fusion protein coding sequence. Said gene construct is under the control of suitable promoter. Gene construct may be a part of plasmid vector capable of replicating in said microorganism or may be introduced into the genome of said microorganism. Introduction of gene construct may be performed by electroporation.

The secretion signal peptide coding sequence codes for a secretion signal peptide. The secretion signal peptide enables the secretion of protein construct from the cell to the growth medium. Such secretion signal peptides preferably include, but are not limited to signal peptide of Usp45 protein (https://www.ncbi.nlm.nih.gov/protein/300072071; ADJ61471.1 secreted 45 kDa protein precursor [Lactococcus cremoris subsp. cremoris NZ9000; SEQ ID No. 28). In some embodiment, the USP45 cDNA coding sequence can be about 90% or about 80% or about 70% identical to residues 227-310 of SEQ ID No. 16, or residues 131-211 of SEQ ID No. 18, or residues 131-211 of SEQ ID No. 20, or residues 231-314 of SEQ ID No. 24. According to the invention a preferred signal peptide of USP45 has sequence SEQ ID No. 27 MAKKKIISAILMSTVILSAAAPLSGVYA (additional alanine was inserted for cloning purposes).

The promoter that controls the expression of mentioned gene construct may be constitutive or inducible. Constitutive promoters enable continuous protein production but are usually prone to lower level of expression. Inducible promoters are also referred to as controlled promoters. Inducible promoters usually provide higher expression level. Inducible promoters include but are not limited to bacteriocin controlled promoters, carbohydrate controlled promoters, pH controlled promoters, phage promoter Φ31 and temperature controlled promoters. Bacteriocin controlled promoters are controlled by addition of bacteriocins. Such bacteriocins include but are not limited to nisin and pediocin. pH controlled promoters may be P170, induced by accumulation of lactic acid at pH 6.0-6.5, Pgad, induced by chloride ions and F1F0-ATPase promoter, induced by acidification. Temperature controlled promoter may be P2, where inactivation of repression is achieved at temperatures above 40°C. Carbohydrate promoters may be lacA and xylA operons that are induced by changing the carbon source in the growth medium.

In some embodiment the TATk cDNA coding sequence can be about 90% or about 80% or about 70% identical to residues 359-390 of SEQ ID No. 16 or residues 260-292 of SEQ ID No. 18. TAT can be the original TAT peptide having 11 amino acids (TAT11) or a TAT peptide having an additional 16 N-terminal amino acids (TAT28) that are derived from the polylinker of the plasmid used for cloning. In some embodiment, TATK can be a modified version of TAT28. In some embodiment said TATk coding sequence can code for an amino acid sequence comprising or consisting of sequence YARKAARQARA (SEQ ID No. 26).

The TATk fusion protein can optionally contain one or more reporter proteins operatively coupled to the TATk polypeptide. Suitable reporter proteins include, but are not limited to, fluorescent proteins (e.g. green fluorescent protein (GFP), red fluorescent protein (RFP), yellow fluorescent protein (YFP), blue fluorescent protein (BFP), and cyan fluorescent protein (CFP)), beta-galactosidase, luciferase (bacterial, firefly, and renilla luciferase), antibiotic-resistance genes (e.g. chloramphenicol acetyltransferase, neomycin phosphotransferase, and NPT-II), p-glucuronidase, and alkaline phosphatase. Inclusion of a reporter protein allows, inter alia, for direct and/or indirect characterization of the fusion protein and function of the fusion protein, as well as affinity purification of the protein. The reporter protein can be operatively linked to the N-terminus and/or the C-terminus of the TATk polypeptide. In other embodiments the reporter protein can be operatively linked to N-terminus and/or the C-terminus of the TATk fusion protein. A TATk fusion protein cDNA according SEQ ID NO: 16 or SEQ ID No. 18 or SEQ ID No. 22 or SEQ ID No. 24 and having an amino acid sequence according to SEQ ID NO: 17 or SEQ ID No. 19 or SEQ ID No. 23 or SEQ ID No. 25, respectively, demonstrate non-limiting embodiments of a TATk fusion protein containing a fluorescent reporter protein. In one or more embodiments, the TATk fusion protein comprises one or more affinity-tags. The affinity-tag is located on one or more of the C-terminus of the TATk fusion protein. Examples of affinity-tags that can be added to the construct include, but are not limited to, epitope tags (e.g. MYC, HA, V5, NE, Strepll, Twin-Strep-tag^{®}, HPC4), 5 glutathione S-transferase (GST), maltose-binding protein (MBP), calmodulin-binding peptide (CBP), FLAG^{®}, 3xFLAG^{®}, polyhistidine (His), and combinations thereof.

### Formulations and uses thereof

A genetically modified non-pathogenic bacterium according to the invention can be used as medicament for the administration of therapeutic molecules for the treatment of rare diseases, in particular for monogenic neurological disorders. In particular the modified lactic acid bacterium of the invention can be used to produce and deliver recombinant TATk-CDKL5 protein in the treatment of CDKL5 deficiency disorder (CDD), and even for other rare CNS pathologies.

Another embodiment of the present invention is the pharmaceutical composition comprising the genetically modified bacteria of the invention in a pharmaceutically effective amount. Preferably pharmaceutical composition, comprising TATk-fusion protein displaying lactic acid bacteria is for oral administration. The pharmaceutical composition may be liquid, comprising lactic acid bacterium expressing TATk-fusion protein, or it may be solid, comprising dried microorganism that can be reactivated when put in a suitable environment. Lactic acid bacteria may be dried by any system, including freeze drying and spray drying. "TATk-fusion protein secreting lactic acid bacterium" as used here, means that the lactic acid bacterium is viable and can express TATk-fusion protein when placed in a suitable environment, not necessarily expressing the TATk-fusion protein in the pharmaceutical composition. The pharmaceutical composition may further comprise agents known to the person skilled in the art to improve viability of the lactic acid bacteria, such as but not limited to skimmed powdered milk.

The pharmaceutical composition further includes a pharmaceutically acceptable carrier. Suitable pharmaceutically acceptable carriers include, but are not limited to, water, salt solutions, alcohols, gum arabic, vegetable oils, polyethylene glycols, gelatin, carbohydrates such as lactose, amylose or starch, magnesium stearate, talc, silicic acid, viscous paraffin, perfume oil, fatty acid esters, hydroxy methylcellulose, and polyvinyl pyrrolidone, which do not deleteriously react with the active composition. The pharmaceutical composition can be mixed with auxiliary agents, such as lubricants, stabilizers, wetting agents, emulsifiers, salts for influencing osmotic pressure, buffers, coloring, flavoring and/or aromatic substances, and the like which do not deleteriously react with the active composition.

In addition to the therapeutically effective amount of the non-pathogenic bacteria described herein, the pharmaceutical composition can also include an effective amount of an auxiliary active agent.

The pharmaceutical compositions contain a therapeutically effective amount of the non-pathogenic bacteria of the invention, and optionally, a therapeutically effective amount of an auxiliary agent in a dosage form. The dosage forms can be adapted for administration by any appropriate route. Appropriate routes preferably include, but are not limited to, oral (including buccal or sublingual). Such formulations may be prepared by any method known in the art.

Dosage forms adapted for oral administration can be discrete dosage units such as capsules, pellets or tablets, powders or granules, solutions, or suspensions in aqueous or nonaqueous liquids; edible foams or whips, or in oil-in-water liquid emulsions or water-in-oil liquid emulsions. In some embodiments, the pharmaceutical formulations adapted for oral administration also include one or more agents which flavor, preserve, color, or help disperse the pharmaceutical formulation. Dosage forms prepared for oral administration can also be in the form of a liquid solution that can be delivered as foam, spray, or liquid solution. In some embodiments, the oral dosage form can contain about 1 ng to 1000 g of a pharmaceutical formulation containing a therapeutically effective amount or an appropriate fraction thereof of non-pathogenic bacteria or composition containing the non-pathogenic bacteria of the invention. The oral dosage form can be administered to a subject in need thereof.

Where appropriate, the dosage forms described herein can be microencapsulated. The dosage form can also be prepared to prolong or sustain the release of any ingredient. Delayed release dosage formulations can be prepared as described in standard references such as "Pharmaceutical dosage form tablets," eds. Liberman et. al. (New York, Marcel Dekker, Inc., 1989), "Remington - The science and practice of pharmacy", 20th ed., Lippincott Williams & Wilkins, Baltimore, MD, 2000, and "Pharmaceutical dosage forms and drug delivery systems", 6th Edition, Ansel et al, (Media, PA: Williams and Wilkins, 1995). These references provide information on excipients, materials, equipment, and processes for preparing tablets and capsules and delayed release dosage forms of tablets and pellets, capsules, and granules. The delayed release can be anywhere from about an hour to about 3 months or more.

Examples of suitable coating materials include, but are not limited to, cellulose polymers such as cellulose acetate phthalate, hydroxypropyl cellulose, hydroxypropyl methylcellulose, hydroxypropyl methylcellulose phthalate, and hydroxypropyl methylcellulose acetate succinate; polyvinyl acetate phthalate, acrylic acid polymers and copolymers, and methacrylic resins that are commercially available under the trade name EUDRAGIT^{®} (Roth Pharma, Westerstadt, Germany), zein, shellac, and polysaccharides.

The non-pathogenic bacteria and pharmaceutical formulations thereof described herein can be used in the protein replacement therapy for the treatment and/or prevention of a disease, disorder, syndrome, or a symptom thereof in a subject. In some embodiments, the non-pathogenic bacteria and pharmaceutical formulations thereof can be used to treat and/or prevent rare monogenic disease including CDKL5deficiency disorder (CDD), Rett syndrome, variants of Rett syndrome, and/or a symptom thereof, Fragile-X syndrome, Lysosomal storage diseases, Claes-Jensen type (MRXSCJ); CASK-related disorders; a 1-antitrypsin deficiency; Kartagener syndrome; Chronic Granulomatous Disease; Xeroderma Pigmentosum, Gaucher disease, Pompe disease, ADA deficiency.

An amount of the non-pathogenic bacteria and pharmaceutical compositions thereof described herein can be administered to a subject in need thereof one or more times per day, week, month, or year. In some embodiments, the amount administered can be the therapeutically effective amount of the non-pathogenic bacteria and pharmaceutical compositions. For example, the non-pathogenic bacteria and pharmaceutical compositions thereof can be administered in a daily dose. This amount may be given in a single dose per day. In other embodiments, the daily dose may be administered over multiple doses per day, in which each containing a fraction of the total daily dose to be administered (sub-doses). In some embodiments, the amount of doses delivered per day is 2, 3, 4, 5, or 6. In further embodiments, the compounds, formulations, or salts thereof are administered one or more times per week, such as 1, 2, 3, 4, 5, or 6 times per week. Lactic acid bacteria thrive in nutrient rich environments such as milk. They are integral part of mammalian G! microbiota and are considered beneficial to human health. As such they are added to the diet in the form of probiotic food. The present invention also relates to a food containing such recombinant lactic acid bacteria. Food composition may be milk, yogurt, curd, cheese, fermented milks, milk based fermented products, ice cream, fermented cereal based products and milk-based powders.

### MATERIALS AND METHODS

### Bacterial strains, media, and growth conditions

*Lactococcus lactis* NZ9000 (de Ruyter et al., 1996; Mierau and Kleerebezem, 2005) and the resulting recombinant *L. lactis* strains transformed with the different plasmids used in this work were routinely grown at 30°C in M17 liquid medium (Millipore Sigma, Burlington, MA, United States) supplemented with 0.5% glucose (Sigma-Aldrich, St. Louis, MO, United States) (GM17 medium) without agitation, or in plates containing the same medium solidified with 1.5% agar (Carlo Erba, Cornaredo (MI), Italy). To maintain selection pressure on *L. lactis* transformants, 10 µg/ml chloramphenicol (Sigma-Aldrich, St. Louis, MO, United States) was added to the growth medium.

### Molecular cloning and plasmid construction

*Inducible GFP plasmids* - Plasmid pNZ8148 (pSH71 derivative, pNisA, Cm^{r}, nisin-controlled expression; (de Ruyter et al., 1996; Mierau and Kleerebezem, 2005)) harbouring a nisin-promoter (pNisA) was used as a backbone plasmid for cloning. For pNZ-pNisA-USP-TATk-GFP plasmid generation, the sequence encoding the protein transduction domain (TATk peptide) fused to the enhanced green fluorescent protein (eGFP) was PCR-amplified with primers designed for the upstream in-frame addition of the Usp45 sequence ((van Asseldonk et al., 1993), Table 1). The resulting USP-TATk-GFP PCR product was then inserted into the backbone plasmid by means of restriction enzyme digestion (Kpnl and Sacl) and ligation to get the final construct.

*Constitutive GFP plasmids* - For pNZ-pPepN-USP-TATk-GFP plasmid generation, an around-the-horn PCR was performed on the pNZ8148 plasmid to get a backbone vector that was devoid of the Nisin promoter. A Gibson Assembly was then performed for the ligation of the backbone vector with the PCR-amplified aminopeptidase N-promoter (pPepN) and USP-TATk-GFP sequences. For pNZ-pPepN-USP-GFP, an around-the-horn PCR was performed to delete the TATκ sequence from the pNZ-pPepN-USP-TATκ-GFP and the construct was created by Golden Gate Assembly (Engler et al., 2008) using the type-IIS enzyme Bbsl digestion and subsequent ligation. The pNZ-pPepN-USP-TATκ-GFP plasmid was a derivative of the pNZ-pNisA-USP-TATκ-GFP construct in which the NisA inducible promoter was replaced with a PepN constitutive promoter. *Inducible CDKL5 plasmids* - To create the pNZ-pNisA-USP-TATκ-CDKL5-FLAG and pNZ-pNisA-TATκ-CDKL5-FLAG plasmids, the expression cassette encoding the protein transduction domain (TATκ peptide), the human cDNA sequence of the human CDKL5 isoform 1 (hCDKL5₁) protein (Trazzi et al., 2018) and the 3x FLAG epitope sequence at the carboxy-terminal end was PCR amplified and inserted into pNZ8148 via Kpnl and Sacl sites. In the case of pNZ-pNisA-USP-TATκ-CDKL5-FLAG, signal peptide for secretion (Usp45) was added at 5'-end with appropriate primer (Table 1) and PCR product was cloned in the same manner.

Either KOD DNA Polymerase (Merck Millipore) or Herculase II Fusion DNA Polymerase (Thermo Fisher) were used for gene amplifications according to the manufacturers' instructions. Primers were from Eurofins Genomics (Ebersberg, Germany) or Integrated DNA Technologies (Leuven, Belgium) and restriction enzymes from Thermo Scientific or New England Biolabs. NucleoSpin Gel and PCR Clean-up (Macherey and Nagel) was used for isolating DNA fragments from agarose gels. T4 DNA ligase was from New England Biolabs or Metabion. Plasmid DNA was isolated with a NucleoSpin Plasmid kit (Macherey and Nagel), with an additional lysozyme treatment step. Lactococci were transformed with electroporation using a Gene Pulser II apparatus (Biorad) or MicroPulser Electroporation Apparatus (Biorad) as described in (Holo and Nes, 1995). Plasmids were sequenced by Eurofins Genomics (Ebersberg, Germany). All primers are listed in Table 1.

**Table 1**

| **pNZ-pNisA-USP-TATk-GFP** | |
|---|---|
| **Primer name (SEQ ID No.)** | **Primer sequence** |
| TAT-GFP-Sacl-REV (SEQ ID No. 1) | TATTGAGCTCTCAATGATGATGATGATGATGGTCGACGGC |
| USP-TAT-Kpnl-FW (SEQ ID No. 2) | TATTGGTACCTTATGGCTAAAAAAAAGATTATCTCAGCTATTTTAATGTCTA- CAGTGA- TACTTTCTGCTGCAGCCCCGTTGTCAGGTGTTTACGCTGACGCGGCCCAGCCG GCCAG |
| USP-Kpnl-FW (SEQ ID No. 3) | TATTGGTACCTTATGGCTAAAAAAAAGATTATCTCAG |
| | |

| **pNZ-pPepN-USP-TATk-GFP** | |
|---|---|
| **Primer name (SEQ ID No.)** | **Primer sequence** |
| pPepN-FW (SEQ ID No. 4) | TACAGCTCCACTCTGTAAAAGCTGTCAG |
| pPepN-REV (SEQ ID No. 5) | TTTTAGCCATATCTTCTCCTAAATATTCAGTATTAATATAATTATATC |
| USP-FW (SEQ ID No. 6) | AGGAGAAGATATGGCTAAAAAAAAGATTATCTCAG |
| TAT-GFP-REV (SEQ ID No. 7) | TTTTGGTTCAATTCAGATCCTCTTCTGAGATGAGTTTTTGTTCGGGCCCTCCTC |
| pNZ-FW (SEQ ID No. 8) | GGATCTGAATTGAACCAAAATTAGAAAACCAAG |
| pNZ-REV (SEQ ID No. 9) | TTTTACAGAGTGGAGCTGTAATATAAAAACC |

| **pNZ-pPepN-USP-GFP** | |
|---|---|
| **Primer name (SEQ ID No.)** | **Primer sequence** |
| GFP-Bbsl-FW (SEQ ID No. 10) | GTACGAAGACCACGCTGTGAGCAAGGGCGAGGAG |
| USP-Bbsl-REV (SEQ ID No. 11) | GCGCGAAGACGCAGCGTAAACACCTGACAACGG |
| | |

| **pNZ-pNisA-TATk-CDKL5-FLAG** | |
|---|---|
| **Primer name (SEQ ID No.)** | **Primer sequence** |
| CDK-F-Kpn (SEQ ID No. 12) | TATTGGTACCTTATGGAGACAGACACACTCCTGC |
| CDK-R-Sac (SEQ ID No. 13) | TATTGAGCTCCTACTTGTCATCGTCATCCTTGTAGTCG |
| | |

| **pNZ-pNisA-USP-TATk-CDKL5-FLAG** | |
|---|---|
| **Primer name (SEQ ID No.)** | **Primer sequence** |
| CDK-USP-F-Kpn-OE CDK-R-Sac (SEQ ID No. 14) | TATTGGTACCTTATGGCTAAAAAAAAGATTATCTCAGCTATTTTAATGTCTA- CAGTGA- TACTTTCTGCTGCAGCCCCGTTGTCAGGTGTTTACGCTATGGAGACAGACACAC TCCTGC |
| USP-F-Kpn CDK-R-Sac (SEQ ID No. 15) | TATTGGTACCTTATGGCTAAAAAAAAGATTATCTCAG |

### Evaluation of GFP protein expression and secretion

Overnight cultures of *L*. *lactis* harbouring the pNZ-pNisA-USP-TATk-GFP, pNZ-pPepN-USP-TAT-GFP, or pNZ-pPepN-USP-GFP plasmids were diluted (1:50) in GM-17 medium supplemented with chloramphenicol, and grown under constant agitation at 30°C, according to different protocols. *L. lactis* transformed with the inducible construct (pNZ-pNisA-USP-TATk-GFP) was grown for 4 hours to reach the exponential growth phase (OD₆₀₀ ≃0.5 - 0.8) and protein expression was induced with nisin (25 ng/mL, Sigma-Aldrich, St. Louis, MO, United States) for an additional incubation time of 3 hours. *L. lactis* strains transformed with the constitutive GFP constructs (pNZ-pPepN-USP-TAT-GFP or pNZ-pPepN-USP-GFP) or with pNZ8148, as a negative control, were grown for 7 hours. For all strains, bacteria culture samples were collected after 4 and 7 hours of growth by centrifugation (5,000×g for 10 min). Pellets were resuspended in PBS for GFP fluorescence measurements. The growth media were transferred to a Vivaspin^{®} Turbo15 Filter column (Sartorius) with 10 kDa molecular weight cut-off, to obtain a 50/100-fold medium concentration according to the manufacturer's instructions. An aliquot of cell pellets and concentrated culture media was stored at -20°C for Western blot analysis.

*GFP fluorescence measurement* - GFP fluorescence from two biological replicates for each strain was measured in triplicates by dispensing 100 ul of PBS-resuspended pellet per well of a 96-Well Black/Clear bottom plate. Fluorescence GFP signals were measured using a GloMax Discover Microplate Reader (Promega, Madison, Wl, USA) with excitation/emission at 488/509 nm.

*Western blot* - Concentrated culture medium and pellet samples were thawed in ice and pellet resuspensions were briefly sonicated (High intensity ultrasonic processor VC100 - 100 watt, Sonics & Materials, inc.). Samples were mixed with 4× Laemmli sample buffer and β-mercaptoethanol, and boiled at 100°C before loading. Equivalent amounts of *L. lactis* extracts and culture media were subjected to electrophoresis on a Bolt^{™} 4-12% Bis-Tris Plus gel (Life Technologies Corporation, Carls- bad, CA, USA) and transferred to a Hybond ECL nitrocellulose membrane (GE Healthcare Bio-Science, Piscataway, NJ, USA). An anti-GFP primary antibody (rabbit polyclonal anti-GFP, 1:1000, Thermo Fisher Scientific, Waltham, MA, USA) and HRP-conjugated goat anti-rabbit IgG secondary antibody (1:5000; Jackson ImmunoResearch Laboratories, West Grove, PA, USA) were used. Images were acquired using a ChemidocTM XRS+ Imaging System and the Image LabTM Software (Bio-Rad, Hercules, CA, USA).

### Determination of bacterial growth and vitality

Overnight culture of *L. lactis* harboring the pNZ-pPepN-USP-TATk-GFP plasmid was diluted (1:50) in fresh GM-17 medium supplemented with chloramphenicol and grown for 8 hours at 30°C under constant agitation. At 1-hour intervals, optical density measurement using a colorimetric spectrophotometer, and serial dilution plating were performed as an indirect and direct method of bacterial viability evaluation, respectively. At each time point, OD was measured by reading the optical density at 600 nm, while colony-forming units (CFUs) were measured by spreading aliquots of a 1:10 dilution series of the batch culture on GM17 agarose plates and then counting formed colonies after 48 hours of growth at 30°C. The measurements were plotted as a function of time to obtain the OD-growth curve and the CFU/mL-growth curve. Finally, to use optical density to approximate bacterial cell density, a standard curve of CFU/mL versus OD₆₀₀ was created, showing a linear regression relating to these units.

### Mouse colony

Adult (3-6-month-old) C57BL/6J mice were used. All mice were housed three to five per cage and maintained in a temperature- (23 °C) and humidity-controlled environment with a standard 12 h light/dark cycle, and were provided with standard mouse chow and water ad libitum. The animals' day-to-day health and comfort were controlled by the veterinary service. All the experiments were conducted in accordance with the Italian and European Community law for the use of experimental animals and with the approval of the National Bioethical Committee (approval number: 195/2023-PR).

### In vivo administration of L. lactis

Overnight cultures of *L. lactis* NZ9000 harboring plasmids pNZ-pPepN-USP-TATκ-GFP or pNZ-pPepN-USP-GFP were diluted (1:50) in fresh GM-17 medium supplemented with chloramphenicol, and grown under constant agitation at 30°C for 6-8 hours to reach the stationary phase, corresponding to an optical density of 1.8-2 measured at a wavelength of 600 nm (OD₆₀₀) using a GENESYS^{™} 10UV Spectrophotometer (Thermo Scientific, Waltham, MA, USA). The culture was then centrifuged at 5000 ×g for 10 min and the pellet resuspended as follows.

For oral delivery through gavage, the pellet was resuspended in phosphate buffered saline (PBS; 10mM Na2HPO4, 1.8mM KH2PO4, 137mM NaCl, and 2.7 mM KCI; pH 7.4) at a concentration of 2 × 10¹¹ CFU/mL and stored at 4 °C until used for oral administration (max. 3 days). To deliver *L*. *lactis* into the stomach, oral gavage was performed with 250 µl of live bacterial suspension in PBS at a dose of 5 × 10¹⁰ CFU/mouse, using a flexible plastic feeding tube (20 gauge, 38 mm) mounted on an insulin syringe (volume: 1,0 ml) following the IACUC Standard Procedure.

For oral delivery in drinking water, the pellet was resuspended in M17, supplemented with chloramphenicol and diluted 1:10 in filtered drinking water, at a concentration of 2 × 10¹⁰ CFU/mL. The pH of the solution was raised to pH 10 to counteract bacterial flocculation caused by metabolism-dependent acidification. Drinking solution containing *L. lactis* was replaced every 3-4 days with freshly cultured bacteria.

### Sample collection for analyses

For intestinal colonization studies, stool samples were collected in PBS and mice were sacrificed immediately afterward to collect bowel contents. For immunohistochemistry studies, mice were deeply anesthetized with isoflurane (2% pure oxygen) and transcardially perfused with PBS to remove blood. Different tracts of the intestine (cecum, ileum, and colon), the liver, and the brain were quickly removed. Intestinal contents were squeezed out and quickly washed in PBS to remove residual content before fixation. All tissues were fixed by immersion in 4% paraformaldehyde in 0.1 M PBS for 24 hours (for intestine) or 48 hours (for liver and brain), kept in 15-20% sucrose for an additional 24-48 hours, frozen with dry ice, and stored at - 80°C.

*Survival and intestinal colonization of L. lactis* - Stool samples and bowel contents resuspended in PBS were vortexed vigorously, briefly centrifuged (4 s. spin) and an aliquot of the supernatant was plated on chloramphenicol-containing GM-17 plates using spread-plate method. Ten-fold dilutions of the caecum supernatant were plated on chloramphenicol-containing GM-17 plates using the drop-plate method (Holo and Nes, 1995) for cell counting. Plates were incubated at 30°C for 48 hours. The number of colony forming units (CFUs) arising after a 48 h growth within the drop area was plotted as a function of time after oral administration of bacteria.

*Immunohistochemistry procedure* - Liver and intestinal tissues were cut with a cryostat (Histo-Line Laboratories) into 15-µm-thick sections, which were mounted onto superfrost slides, air-dried, and then processed for immunohistochemistry or stored at -20°C. Brain tissues were cut with a freezing microtome (Microm GmbH, Walldorf, Germany) into 30-µm-thick coronal sections which were serially collected in 96-well plates containing a solution composed of 30% glycerol, 30% ethylene glycol, and 0.02% sodium azide in 0.1 M PBS, and then processed for immunohistochemistry or stored at -20°C.

Tissue sections were incubated overnight at 4°C with a primary anti-GFP antibody (rabbit polyclonal anti-GFP, 1:200, Thermo Fisher Scientific), and then for 2 hours with an HRP-conjugated anti-rabbit secondary antibody (1:200, Jackson Immunoresearch, West Grove, PE, USA). Detection was performed using the TSA Cyanine 3 Plus Evaluation Kit (Perkin Elmer, Waltham, MA, USA). Fluorescent images, two images per section, were acquired using a Nikon Eclipse TE600 microscope equipped with a Nikon Digital Camera DXM1200 ATI System (Nikon Instruments Inc. Tokyo, Japan). For intensity-based analysis of GFP immunoreactivity all images were acquired with the same gain and exposure time. The fluorescence signal intensity of GFP staining in different tissues (caecum, liver, cortex and hippocampus) was quantified starting from 20x magnification images of GFP-stained tissue slices. The average intensities of the fluorescent signals were quantified by determining the number of positive (bright) pixels within three randomly selected areas. A total of six slices were evaluated for each sample. Mean optical intensity data are given as percentages in relation to vehicle treated mice.

### Statistical analyses

Statistical analysis was performed using GraphPad Prism 8.0.1 (GraphPad Software, Boston, MA, USA). Values are expressed as means ± standard error (SEM). The significance of results was obtained using the two-tailed Student's t-test or one-way analysis of variance (one-way ANOVA) followed by Fisher's LSD post hoc test, as specified in the figure legends. A probability level of p<0.05 was considered statistically significant. The confidence level was taken as 95%.

### EXAMPLES

### Example 1 - Production of constructs for TATk-GFP protein expression in L. lactis

To obtain the proof of concept that a TATk-fusion protein secreted by *L. lactis* in the intestine is transduced by the intestinal epithelium, delivered into the circulation, and subsequently reaches different organs, including the brain, the inventors used a recombinant *L. lactis* to produce and secrete the TATk-GFP protein.

For recombinant TATk protein expression in *L. lactis* two strategies were adopted: inducible and constitutive gene expression. TATκ-GFP gene was cloned either into the pNZ8148 plasmid which contains an inducible Nisin promoter (pNZ-pNisA), or into the pNZ8148 plasmid into which the constitutive pPepN promoter was introduced in the place of the inducible Nisin promoter (pNZ-pPepN). Moreover, in order to induce TATκ-GFP protein secretion, the signal peptide for secretion in *L. lactis,* Usp45 (Plavec and Berlec, 2019), was cloned at the 5'-terminal of the genes. A GFP construct with the Usp45 secretion peptide but without the TATk sequence was created as a control for TATκ-GFP protein transduction and therefore for *in vivo* tissue biodistribution. The inducible and constitutive TATk-GFP and the constitutive GFP plasmids are reported in Figure 2.

### Example 2 - Quantification of TATk-GFP protein expression and secretion

GFP fluorescence measurement can be used as a direct index of TATk-GFP protein expression. To evaluate which plasmid, inducible or constitutive, was more effective in the expression and secretion of the TATk-GFP protein, fluorescence intensity of the *L. lactis* culture or growth medium was quantified using a fluorescence microplate reader. Overnight cultures of *L. lactis* harboring TATk-GFP plasmids were diluted 1:50 in M-17 medium supplemented with glucose and chloramphenicol, and grown at 30°C, according to different protocols, as follows: *L*. *lactis* transformed with the inducible construct pNZ-pNisA-USP-TATk-GFP was grown to an OD₆₀₀ of 0.5 (an exponential growth phase) and TATk-GFP protein expression was induced using nisin (25 ng/mL) during an additional incubation time of 3 hours (Fig. 3A). Separately, *L. lactis* transformed with the constitutive TATk-GFP and GFP constructs (pNZ-pPepN-USP-TATk-GFP and pNZ-pPepN-USP-GFP) was diluted 1:50 after overnight culture, and grown at 30°C for the time indicated in Figure 3A. Samples of *L. lactis* were collected after reaching an OD₆₀₀ of 0.5, or 3 hours later. As a negative control, *L. lactis* transformed with the empty plasmid pNZ-pNisA was grown in the same condition. At the end of the growing protocol, bacterial cultures were harvested by centrifugation, separated from the growth media, and resuspended in the PBS to a final OD₆₀₀ of 4. Fluorescence was measured using a microplate reader, with excitation/emission at 488/509 nm. A schematic representation of the different protocols of growth and the measuring instrument is shown in Figure 3A.

Concerning fluorescence of bacterial cell resuspension, similar fluorescence intensity was found in *L. lactis* harboring the pNZ-pPepN-USP-TATk-GFP or the pNZ-pPepN-USP-GFP plasmid upon reaching OD₆₀₀ 0.5 (Fig. 3B, 4h), indicating same efficiency of expression of the TATk-GFP and GFP proteins by *L. lactis* (Fig. 3B). As expected, without stimulation from nisin the fluorescence intensity of *L. lactis* harboring the pNZ-pNisA-USP-TATk-GFP was not different from the background levels (pNZ-pNisA empty plasmid, Fig. 3B). Three hours after induction with nisin, the fluorescence intensity in *L. lactis* harboring the pNZ-pNisA-USP-TATk-GFP plasmid increased, reaching levels that were even higher than those achieved with the constituent pNZ-pPepN-USP-TATk-GFP plasmid (Fig. 3B, 7h).

Concerning the culture medium, high background fluorescence was present in all the collected media. To get rid of this unspecific fluorescence that might mask the intrinsic fluorescence given off by the secreted TATk-GFP protein, culture media were concentrated, and a buffer exchange with PBS was performed. Unfortunately, it was not possible to measure a fluorescence intensity over the background (growth medium of *L. lactis* carrying an empty plasmid) in the culture media of *L. lactis* harboring either the constitutive or the inducible plasmid.

To confirm secretion of the TATk-GFP protein by the secretion peptide Usp45, protein extracts from *L. lactis* (4h and 7h) and samples of growth medium were analyzed using Western blot. Confirming results obtained through fluorescence measurement (Fig. 3), similar expression levels were found of TATk-GFP and GFP proteins in *L. Lactis* harboring the pNZ-pPepN-USP-TATk-GFP or pNZ-pPepN-USP-GFP plasmid (Fig. 4, 4h and 7h, *L. lactis extract*)*,* and a slightly greater expression of TATκ-GFP in *L. lactis* harboring the inducible plasmid (Fig. 4, 7h, *L. lactis extract*)*.* Interestingly, the secreted TATk-GFP protein was detectable only in the concentrated growth medium of *L. lactis* harboring the constitutive pNZ-pPepN-USP-TATk-GFP plasmid and not in *L. lactis* harboring the inducible plasmid (Fig. 4, *growth medium*)*.* The lack of TATk-GFP detection in the culture medium after induction with nisin could be attributed to the time of induction (3h; Fig. 4 line 6, *growth medium*)*,* that may not have been enough to induce an accumulation of TATk-GFP protein in the culture medium that would then be detectable by Western blot analysis. Confirming efficient protein secretion by constitutive expression, GFP protein was similarly detectable in the concentrated growth medium of *L. lactis* harboring the constitutive pNZ-pPepN-USP-GFP plasmid (Fig. 4, *growth medium*)*.*

This finding confirms that the Usp45 peptide drives the secretion of the TATk-GFP protein from *L. lactis,* and indicates that constitutive TATk-GFP expression is more effective in inducing an enrichment of recombinant protein in the medium.

Based on these results, for in vivo studies of live production of secretable TATk-GFP protein, recombinant *L. lactis* harboring the constitutive expression plasmid pNZ-pPepN-USP-TATk-GFP was used.

### Example 3 - Effect of TATk-GFP protein expression on L. lactis viability

To test whether constitutive TATk-GFP expression, due to the presence of the high charge TATk peptide, affects *L. lactis* growth, a quantitative detection of the bacterial population was carried out through spectrophotometric analysis. *L. lactis* harboring the pNZ-pPepN-USP-TATk-GFP plasmid was grown for 8 hours. Optical density (OD) at 600 nm (OD₆₀₀), as a measure of the bacterial cell density, was measured every hour. Since the spectrophotometric method estimates total cell biomass including both live and dead cells, in order to test whether constitutive TATk-GFP expression affects *L. lactis* viability we performed a colony formation assay to evaluate the total number of live *L. lactis.* A ten-fold dilution of the cultures was plated onto GM-17 plates containing 10 µg/mL chloramphenicol at the same time as the OD measurement. The number of colonies was counted after 2 days of incubation at 30°C. Overall, the growth of *L. lactis* harboring the pNZ-pPepN-USP-TATk-GFP plasmid, shown as OD at each time point (Fig. 5A), had a regular sigmoidal growth pattern with a lag phase, a log phase, and a stationary phase, after which the number of dead cells exceeds that of living cells. The stationary phase begins after 7-8 hours, when the bacterial culture reaches an OD₆₀₀ of 2 (Fig. 5A). The viability of the same culture was assessed by counting the number of colony forming units (CFUs), and expressed as CFU/mL as a function of time (Fig. 5B). It was found that constitutive TATk-GFP expression did not impair the growth or viability of *L. lactis.*

A standard curve (a linear regression) for CFU/mL versus OD₆₀₀ was plotted and used to provide an approximation of the CFU/mL of the *L. lactis* culture based on the OD measurement (Fig. 5C).

### Example 4 - Efficacy and persistence of intestinal colonization of recombinant L. lactis and in vivo expression of TATk-GFP protein

To explore the survival and colonizing properties of recombinant *L. lactis* in vivo, adult (6-month-old) mice were orally administered with 5×10¹⁰ CFU of *L. lactis* harboring pNZ-pPepN-USP-TATk-GFP plasmid, or PBS as a control. After 2h, 4h, 8h, 16h, 24h, and 32h, stool samples and bowel contents were collected to evaluate the intestinal presence of recombinant *L. lactis* (Fig. 6A). After 2 h, we found fluorescent *L. lactis* in frozen thin sectioned gastrointestinal tract samples (Fig. 6B), indicating that *L. lactis* continues to express the TATk-GFP protein in the host organism. Concerning the colonizing properties of *L. lactis,* the presence of viable *L. lactis* in stool samples and bowel contents was estimated by its ability to form colonies after plating. We found that the maximum concentration of viable *L. lactis* in the intestine was reached 2-4 hours after gavage. Sixteen hours later, the amount of *L. lactis* remaining in the intestine was very low compared to 2 hours after gavage (Fig. 6C, D), and undetectable after 36 hours, indicating that a single oral administration does not allow for a prolonged *L. lactis* intestinal colonization. On the other hand, after three administrations, interspaced by 2 hours (Fig. 6E), the inventors found high levels of recombinant *L. lactis* in the intestinal tract, including the ileus, 20 hours after the last administration (Fig. 6F), suggesting that repeated treatments lead to more persistent gut colonization throughout the intestine.

### Example 5 - Evaluation of the secretable TATk-GFP protein biodistribution in mice

To evaluate the efficiency of the *in vivo* TATk-GFP protein secretion and effectiveness in biodistribution, the inventors used a repeated treatment schedule either during the same day (Fig. 7A) or on successive days (2 days, Fig. 8A; 4 days).

### Intestinal TATk-GFP protein biodistribution

It was first evaluated whether secreted TATk-GFP is internalized by the intestinal epithelium. Two (at 6h) or twenty (at 24h) hours after receiving three doses of *L. lactis* (Fig. 7A) mice were sacrificed, the intestine was washed to remove the contents and fixed before being immunostained using an anti-GFP antibody. Higher GFP immunofluorescence intensity was found in the intestinal wall of mice treated with recombinant *L. lactis* (Fig. 7B,C), indicating that intestinal cells were transduced by secreted TATk-GFP. Interestingly, similar, if not greater, levels of intestinal TATk-GFP 20 hours (24h) were found after the last *L. lactis* gavage to those of 2 hours (6h), confirming that a repeated daily treatment prolongs *L. lactis* gut colonization (Fig. 7C).

### Liver TATk-GFP protein biodistribution

After two days of treatment (Fig. 8A), TATk-GFP protein was immunodetected not only in the intestinal wall (Fig. 8B,D) but also in the liver (Fig. 8C,D), suggesting that the secreted TATk-GFP protein reaches the liver through the portal circulation and transduces hepatocytes.

No GFP staining over control background was detectable in the intestinal or liver tissue samples of mice treated with *L. lactis* harboring the control plasmid, pNZ-pPepN-USP-GFP (Fig. 8B-D), confirming that only a TATk-fusion protein transduces the intestinal epithelium and subsequently reaches other tissues/organs through the circulation.

### Brain TATk-GFP protein biodistribution

After the four-day treatment schedule with recombinant TATk-GFP *L. lactis,* it was detected higher TATk-GFP immunofluorescence, not only in the intestine and liver of treated mice in comparison with vehicle-treated mice (Fig. 9), but also in the brain (Fig. 10), suggesting that the TATk-GFP protein passed the blood-brain barrier and reached the brain through the bloodstream.

By comparing the TATk-GFP fluorescence intensity in mouse tissues after 2 and 4 days of treatment, no difference was found in TATk-GFP fluorescence intensity in the mouse intestine and liver (Fig. 11). Differently, at the brain level the fluorescence intensity for TATk-GFP was higher after 4 days of treatment, indicating increased brain TATk-GFP protein delivery as a function of treatment time, and suggesting that prolonged treatment may have higher therapeutic efficacy for/in the brain.

These results provide the first evidence that a TATk fusion protein, secreted by *L. lactis* in the intestine, can efficiently pass from the intestine to other organs thanks to the transduction properties of the TATk peptide.

### Example 6 - Oral delivery of L. lactis in drinking water: efficient L. lactis intestinal colonization and secretable TATk-GFP protein biodistribution

Since protein therapy for genetic diseases could be a lifelong treatment, with daily administration, a prolonged treatment in mice must be envisaged to evaluate the therapeutic/safety efficacy of this approach. Oral delivery of *L. lactis* in drinking water is the most convenient administration strategy as it does not provoke handling stress for mice and allows a continuous supply of *L. lactis* during the day. To explore whether the administration with drinking water allows for a sufficient concentration of *L. lactis* at the intestinal level to detect the secreted TATk-GFP protein at the tissue level, mice were chronically treated (7 days; Fig. 12A) with L. lactis in drinking water. Daily mean water intake in adult mice was calculated to be 5.0 ± 0.2 ml/mouse (Bachmanov et al., 2002). To mimic the dose of *L. lactis* that was administrated during the twice-daily oral gavage, 1 × 10¹¹ *L. lactis* were resuspended in 5 ml of water. High levels of recombinant *L. lactis* were found in the intestinal tract at the end of the 7-day treatment (Fig. 12A), indicating that the *L. lactis* was able to form colonies after the bowel contents were plated (Fig. 12A), and higher GFP immunofluorescence intensity in the intestinal wall of mice treated with recombinant *L. lactis* (Fig. 12B,C), indicating that oral delivery of *L. lactis* in drinking water is sufficient to produce and maintain a high intestinal *L. lactis* level.

### Example 7 - Engineering of L. lactis for delivery of therapeutic TATk-CDKL5 protein

Inventors recently provided novel evidence that a protein therapy, aimed at compensating for the lack of CDKL5 function in the brain, is feasible, and effective at rescuing the neurological phenotypes of a mouse model of CDD (Trazzi et al., 2018). Despite the potential effectiveness of a protein replacement approach, manufacturing turned out to be a problem for the recombinant CDKL5 protein; its purification in diverse biological matrices such as bacteria, and insect and mammalian cells was ineffective due to low CDKL5 protein solubility outside the physiological environment, resulting in high/unsustainable production costs.

Since it has not been possible, or is very difficult, to produce full-length CDKL5 protein in bacteria thus far (Calvanese et al., 2022; Katayama et al., 2015), an initial assessment of its production in *L. lactis* was carried out. An expression plasmid driving TATk-CDKL5 expression, pNZ-pNisA-TATκ-CDKL5-FLAG, was constructed (Fig. 13A,B). Efficient TATk-CDKL5 full length expression was confirmed through Western blot analysis on *L. lactis* cell extracts, using an anti-FLAG antibody (Fig. 13D).

Based on this observation a pNZ-pNisA-USP-TATκ-CDKL5-FLAG plasmid driving secretion of TATk-CDKL5 protein into growth medium was constructed (Fig. 13A,C). Secretion of TATk-CDKL5 protein into the growth medium, in comparison to the control, was observed (Fig. 13E), and indicated that *L. lactis* is an efficient platform for the production/secretion of TATk-CDKL5 protein.

### SEQUENCES

**pNZ-pNisA-USP-TATk-GFP (DNA sequence; SEQ ID No 16)**

| Name | location |
|---|---|
| pNisA | 1.. 184 |
| Usp45 | 227..310 |
| TATk | 359..390 |
| GFP | 404..1117 |
| Myc Tag | 1154..1177 |
| His Tag | 1190..1207 |
| Factor Xa site | rev:1612..1623 |
| repA | 2234..2932 |
| cat | 3391..4041 |

1 CTAGTCTTAT AACTATACTG ACAATAGAAA CATTAACAAA TCTAAAACAG TCTTAATTCT
61 ATCTTGAGAA AGTATTGGTA ATAATATTAT TGTCGATAAC GCGAGCATAA TAAACGGCTC
121 TGATTAAATT CTGAAGTTTG TTAGATACAA TGATTTCGTT CGAAGGAACT ACAAAATAAA
181 TTATAAGGAG GCACTCACCA TGGGTACTGC AGGCATGCGG TACCTTATGG CTAAAAAAAA
241 GATTATCTCA GCTATTTTAA TGTCTACAGT GATACTTTCT GCTGCAGCCC CGTTGTCAGG
301 TGTTTACGCT GACGCGGCCC AGCCGGCCAG GCGCGCGCGC CGTACGAAGC TTGCGGCCTA
361 CGCCAGAAAG GCCGCCAGGC AGGCCAGGGC ACCGGTCGCC ACTGTGAGCA AGGGCGAGGA
421 GCTGTTCACC GGGGTGGTGC CCATCCTGGT CGAGCTGGAC GGCGACGTAA ACGGCCACAA
481 GTTCAGCGTG TCCGGCGAGG GCGAGGGCGA TGCCACCTAC GGCAAGCTGA CCCTGAAGTT
541 CATCTGCACC ACCGGCAAGC TGCCCGTGCC CTGGCCCACC CTCGTGACCA CCCTGACCTA
601 CGGCGTGCAG TGCTTCAGCC GCTACCCCGA CCACATGAAG CAGCACGACT TCTTCAAGTC
661 CGCCATGCCC GAAGGCTACG TCCAGGAGCG CACCATCTTC TTCAAGGACG ACGGCAACTA
721 CAAGACCCGC GCCGAGGTGA AGTTCGAGGG CGACACCCTG GTGAACCGCA TCGAGCTGAA
781 GGGCATCGAC TTCAAGGAGG ACGGCAACAT CCTGGGGCAC AAGCTGGAGT ACAACTACAA
841 CAGCCACAAC GTCTATATCA TGGCCGACAA GCAGAAGAAC GGCATCAAGG TGAACTTCAA
901 GATCCGCCAC AACATCGAGG ACGGCAGCGT GCAGCTCGCC GACCACTACC AGCAGAACAC
961 CCCCATCGGC GACGGCCCCG TGCTGCTGCC CGACAACCAC TACCTGAGCA CCCAGTCCGC
1021 CCTGAGCAAA GACCCCAACG AGAAGCGCGA TCACATGGTC CTGCTGGAGT TCGTGACCGC
1081 CGCCGGGATC ACTCTCGGCA TGGACGAGCT GTACAAGTCC GGACTCAGAT CTCGAGGAGG
1141 GCCCGAACAA AAACTCATCT CAGAAGAGGA TCTGAATAGC GCCGTCGACC ATCATCATCA
1201 TCATCATTGA GAGCTCAAGC TTTCTTTGAA CCAAAATTAG AAAACCAAGG CTTGAAACGT
1261 TCAATTGAAA TGGCAATTAA ACAAATTACA GCACGTGTTG CTTTGATTGA TAGCCAAAAA
1321 GCAGCAGTTG ATAAAGCAAT TACTGATATT GCTGAAAAAT TGTAATTTAT AAATAAAAAT
1381 CACCTTTTAG AGGTGGTTTT TTTATTTATA AATTATTCGT TTGATTTCGC TTTCGATAGA
1441 ACAATCAAAT CGTTTCTGAG ACGTTTTAGC GTTTATTTCG TTTAGTTATC GGCATAATCG
1501 TTAAAACAGG CGTTATCGTA GCGTAAAAGC CCTTGAGCGT AGCGTGCTTT GCAGCGAAGA
1561 TGTTGTCTGT TAGATTATGA AAGCCGATGA CTGAATGAAA TAATAAGCGC AGCGTCCTTC
1621 TATTTCGGTT GGAGGAGGCT CAAGGGAGTT TGAGGGAATG AAATTCCCTC ATGGGTTTGA

1681 TTTTAAAAAT TGCTTGCAAT TTTGCCGAGC GGTAGCGCTG GAAAATTTTT GAAAAAAATT
1741 TGGAATTTGG AAAAAAATGG GGGGAAAGGA AGCGAATTTT GCTTCCGTAC TACGACCCCC
1801 CATTAAGTGC CGAGTGCCAA TTTTTGTGCC AAAAACGCTC TATCCCAACT GGCTCAAGGG
1861 TTTGAGGGGT TTTTCAATCG CCAACGAATC GCCAACGTTT TCGCCAACGT TTTTTATAAA
1921 TCTATATTTA AGTAGCTTTA TTGTTGTTTT TATGATTACA AAGTGATACA CTAATTTTAT
1981 AAAATTATTT GATTGGAGTT TTTTAAATGG TGATTTCAGA ATCGAAAAAA AGAGTTATGA
2041 TTTCTCTGAC AAAAGAGCAA GATAAAAAAT TAACAGATAT GGCGAAACAA AAAGGTTTTT
2101 CAAAATCTGC GGTTGCGGCG TTAGCTATAG AAGAATATGC AAGAAAGGAA TCAGAACAAA
2161 AAAAATAAGC GAAAGCTCGC GTTTTTAGAA GGATACGAGT TTTCGCTACT TGTTTTTGAT
2221 AAGGTAATAT ATCATGGCTA TTAAAAATAC TAAAGCTAGA AATTTTGGAT TTTTATTATA
2281 TCCTGACTCA ATTCCTAATG ATTGGAAAGA AAAATTAGAG AGTTTGGGCG TATCTATGGC
2341 TGTCAGTCCT TTACACGATA TGGACGAAAA AAAAGATAAA GATACATGGA ATAGTAGTGA
2401 TGTTATACGA AATGGAAAGC ACTATAAAAA ACCACACTAT CACGTTATAT ATATTGCACG
2461 AAATCCTGTA ACAATAGAAA GCGTTAGGAA CAAGATTAAG CGAAAATTGG GGAATAGTTC
2521 AGTTGCTCAT GTTGAGATAC TTGATTATAT CAAAGGTTCA TATGAATATT TGACTCATGA
2581 ATCAAAGGAC GCTATTGCTA AGAATAAACA TATATACGAC AAAAAAGATA TTTTGAACAT
2641 TAATGATTTT GATATTGACC GCTATATAAC ACTTGATGAA AGCCAAAAAA GAGAATTGAA
2701 GAATTTACTT TTAGATATAG TGGATGACTA TAATTTGGTA AATACAAAAG ATTTAATGGC
2761 TTTTATTCGC CTTAGGGGAG CGGAGTTTGG AATTTTAAAT ACGAATGATG TAAAAGATAT
2821 TGTTTCAACA AACTCTAGCG CCTTTAGATT ATGGTTTGAG GGCAATTATC AGTGTGGATA
2881 TAGAGCAAGT TATGCAAAGG TTCTTGATGC TGAAACGGGG GAAATAAAAT GACAAACAAA
2941 GAAAAAGAGT TATTTGCTGA AAATGAGGAA TTAAAAAAAG AAATTAAGGA CTTAAAAGAG
3001 CGTATTGAAA GATACAGAGA AATGGAAGTT GAATTAAGTA CAACAATAGA TTTATTGAGA
3061 GGAGGGATTA TTGAATAAAT AAAAGCCCCC CTGACGAAAG TCGACGGCAA TAGTTACCCT
3121 TATTATCAAG ATAAGAAAGA AAAGGATTTT TCGCTACGCT CAAATCCTTT AAAAAAACAC
3181 AAAAGACCAC ATTTTTTAAT GTGGTCTTTA TTCTTCAACT AAAGCACCCA TTAGTTCAAC
3241 AAACGAAAAT TGGATAAAGT GGGATATTTT TAAAATATAT ATTTATGTTA CAGTAATATT
3301 GACTTTTAAA AAAGGATTGA TTCTAATGAA GAAAGCAGAC AAGTAAGCCT CCTAAATTCA
3361 CTTTAGATAA AAATTTAGGA GGCATATCAA ATGAACTTTA ATAAAATTGA TTTAGACAAT
3421 TGGAAGAGAA AAGAGATATT TAATCATTAT TTGAACCAAC AAACGACTTT TAGTATAACC
3481 ACAGAAATTG ATATTAGTGT TTTATACCGA AACATAAAAC AAGAAGGATA TAAATTTTAC
3541 CCTGCATTTA TTTTCTTAGT GACAAGGGTG ATAAACTCAA ATACAGCTTT TAGAACTGGT
3601 TACAATAGCG ACGGAGAGTT AGGTTATTGG GATAAGTTAG AGCCACTTTA TACAATTTTT
3661 GATGGTGTAT CTAAAACATT CTCTGGTATT TGGACTCCTG TAAAGAATGA CTTCAAAGAG
3721 TTTTATGATT TATACCTTTC TGATGTAGAG AAATATAATG GTTCGGGGAA ATTGTTTCCC
3781 AAAACACCTA TACCTGAAAA TGCTTTTTCT CTTTCTATTA TTCCTTGGAC TTCATTTACT
3841 GGGTTTAACT TAAATATCAA TAATAATAGT AATTACCTTC TACCCATTAT TACAGCAGGA
3901 AAATTCATTA ATAAAGGTAA TTCAATATAT TTACCGCTAT CTTTACAGGT ACATCATTCT
3961 GTTTGTGATG GTTATCATGC AGGATTGTTT ATGAACTCTA TTCAGGAATT GTCAGATAGG
4021 CCTAATGACT GGCTTTTATA ATATGAGATA ATGCCGACTG TACTTTTTAC AGTCGGTTTT
4081 CTAATGTCAC TAACCTGCCC CGTTAGTTGA AGAAGGTTTT TATATTACAG CTCCAAGAT

**pNZ-pNisA-USP-TATk-GFP (Amino Acid Sequence; SEQ ID No 17)** 
MAKKKIISAILMSTVILSAAAPLSGVYADAAQPARRARRTKLAAYARKAARQARAPVATVSKGEELFTGVVPILVEL
DGDVNGHKFSVSGEGEGDATYGKLTLKFICTTGKLPVPWPTLVTTLTYGVQCFSRYPDHMKQHDFFKSAMPEGYVQE
RTIFFKDDGNYKTRAEVKFEGDTLVNRIELKGIDFKEDGNILGHKLEYNYNSHNVYIMADKQKNGIKVNFKIRHNIE
DGSVQLADHYQQNTPIGDGPVLLPDNHYLSTQSALSKDPNEKRDHMVLLEFVTAAGITLGMDELYKSGLRSRGGPEQ
KLISEEDLNSAVDHHHHHH-
 **pNZ-pPepN-USP-TATk-GFP (DNA sequence; SEQ ID No 18)**

| Name | location |
|---|---|
| pPepN | 1..130 |
| Usp45 | 131..211 |
| TATk | 260..292 |
| GFP | 305..1018 |
| Myc tag | 1055..1078 |
| Factor Xa site | rev:1464..1475 |
| repA | 2086..2784 |
| cat | 3243..3893 |

1 CTCTGTAAAA GCTGTCAGTA GACAGTTTTT TTAATAAGTT AAAGAAAAGA TGTAATTTTT
61 CTTTGTACTC GAAATTTTCT ATTCAATTTG ATATAATTAT ATTAATACTG AATATTTAGG
121 AGAAGATATG GCTAAAAAAA AGATTATCTC AGCTATTTTA ATGTCTACAG TGATACTTTC
181 TGCTGCAGCC CCGTTGTCAG GTGTTTACGC TGACGCGGCC CAGCCGGCCA GGCGCGCGCG
241 CCGTACGAAG CTTGCGGCCT ACGCCAGAAA GGCCGCCAGG CAGGCCAGGG CACCGGTCGC
301 CACTGTGAGC AAGGGCGAGG AGCTGTTCAC CGGGGTGGTG CCCATCCTGG TCGAGCTGGA
361 CGGCGACGTA AACGGCCACA AGTTCAGCGT GTCCGGCGAG GGCGAGGGCG ATGCCACCTA
421 CGGCAAGCTG ACCCTGAAGT TCATCTGCAC CACCGGCAAG CTGCCCGTGC CCTGGCCCAC
481 CCTCGTGACC ACCCTGACCT ACGGCGTGCA GTGCTTCAGC CGCTACCCCG ACCACATGAA
541 GCAGCACGAC TTCTTCAAGT CCGCCATGCC CGAAGGCTAC GTCCAGGAGC GCACCATCTT
601 CTTCAAGGAC GACGGCAACT ACAAGACCCG CGCCGAGGTG AAGTTCGAGG GCGACACCCT
661 GGTGAACCGC ATCGAGCTGA AGGGCATCGA CTTCAAGGAG GACGGCAACA TCCTGGGGCA
721 CAAGCTGGAG TACAACTACA ACAGCCACAA CGTCTATATC ATGGCCGACA AGCAGAAGAA
781 CGGCATCAAG GTGAACTTCA AGATCCGCCA CAACATCGAG GACGGCAGCG TGCAGCTCGC
841 CGACCACTAC CAGCAGAACA CCCCCATCGG CGACGGCCCC GTGCTGCTGC CCGACAACCA
901 CTACCTGAGC ACCCAGTCCG CCCTGAGCAA AGACCCCAAC GAGAAGCGCG ATCACATGGT
961 CCTGCTGGAG TTCGTGACCG CCGCCGGGAT CACTCTCGGC ATGGACGAGC TGTACAAGTC
1021 CGGACTCAGA TCTCGAGGAG GGCCCGAACA AAAACTCATC TCAGAAGAGG ATCTGAATTG
1081 AACCAAAATT AGAAAACCAA GGCTTGAAAC GTTCAATTGA AATGGCAATT AAACAAATTA
1141 CAGCACGTGT TGCTTTGATT GATAGCCAAA AAGCAGCAGT TGATAAAGCA ATTACTGATA

| | | | | | | |
|---|---|---|---|---|---|---|
| 1201 | TTGCTGAAAA | ATTGTAATTT | ATAAATAAAA | ATCACCTTTT | AGAGGTGGTT | TTTTTATTTA |
| 1261 | TAAATTATTC | GTTTGATTTC | GCTTTCGATA | GAACAATCAA | ATCGTTTCTG | AGACGTTTTA |
| 1321 | GCGTTTATTT | CGTTTAGTTA | TCGGCATAAT | CGTTAAAACA | GGCGTTATCG | TAGCGTAAAA |
| 1381 | GCCCTTGAGC | GTAGCGTGCT | TTGCAGCGAA | GATGTTGTCT | GTTAGATTAT | GAAAGCCGAT |
| 1441 | GACTGAATGA | AATAATAAGC | GCAGCGTCCT | TCTATTTCGG | TTGGAGGAGG | CTCAAGGGAG |
| 1501 | TTTGAGGGAA | TGAAATTCCC | TCATGGGTTT | GATTTTAAAA | ATTGCTTGCA | ATTTTGCCGA |
| 1561 | GCGGTAGCGC | TGGAAAATTT | TTGAAAAAAA | TTTGGAATTT | GGAAAAAAAT | GGGGGGAAAG |
| 1621 | GAAGCGAATT | TTGCTTCCGT | ACTACGACCC | CCCATTAAGT | GCCGAGTGCC | AATTTTTGTG |
| 1681 | CCAAAAACGC | TCTATCCCAA | CTGGCTCAAG | GGTTTGAGGG | GTTTTTCAAT | CGCCAACGAA |
| 1741 | TCGCCAACGT | TTTCGCCAAC | GTTTTTTATA | AATCTATATT | TAAGTAGCTT | TATTGTTGTT |
| 1801 | TTTATGATTA | CAAAGTGATA | CACTAATTTT | ATAAAATTAT | TTGATTGGAG | TTTTTTAAAT |
| 1861 | GGTGATTTCA | GAATCGAAAA | AAAGAGTTAT | GATTTCTCTG | ACAAAAGAGC | AAGATAAAAA |
| 1921 | ATTAACAGAT | ATGGCGAAAC | AAAAAGGTTT | TTCAAAATCT | GCGGTTGCGG | CGTTAGCTAT |
| 1981 | AGAAGAATAT | GCAAGAAAGG | AATCAGAACA | AAAAAAATAA | GCGAAAGCTC | GCGTTTTTAG |
| 2041 | AAGGATACGA | GTTTTCGCTA | CTTGTTTTTG | ATAAGGTAAT | ATATCATGGC | TATTAAAAAT |
| 2101 | ACTAAAGCTA | GAAATTTTGG | ATTTTTATTA | TATCCTGACT | CAATTCCTAA | TGATTGGAAA |
| 2161 | GAAAAATTAG | AGAGTTTGGG | CGTATCTATG | GCTGTCAGTC | CTTTACACGA | TATGGACGAA |
| 2221 | AAAAAAGATA | AAGATACATG | GAATAGTAGT | GATGTTATAC | GAAATGGAAA | GCACTATAAA |
| 2281 | AAACCACACT | ATCACGTTAT | ATATATTGCA | CGAAATCCTG | TAACAATAGA | AAGCGTTAGG |
| 2341 | AACAAGATTA | AGCGAAAATT | GGGGAATAGT | TCAGTTGCTC | ATGTTGAGAT | ACTTGATTAT |
| 2401 | ATCAAAGGTT | CATATGAATA | TTTGACTCAT | GAATCAAAGG | ACGCTATTGC | TAAGAATAAA |
| 2461 | CATATATACG | ACAAAAAAGA | TATTTTGAAC | ATTAATGATT | TTGATATTGA | CCGCTATATA |
| 2521 | ACACTTGATG | AAAGCCAAAA | AAGAGAATTG | AAGAATTTAC | TTTTAGATAT | AGTGGATGAC |
| 2581 | TATAATTTGG | TAAATACAAA | AGATTTAATG | GCTTTTATTC | GCCTTAGGGG | AGCGGAGTTT |
| 2641 | GGAATTTTAA | ATACGAATGA | TGTAAAAGAT | ATTGTTTCAA | CAAACTCTAG | CGCCTTTAGA |
| 2701 | TTATGGTTTG | AGGGCAATTA | TCAGTGTGGA | TATAGAGCAA | GTTATGCAAA | GGTTCTTGAT |
| 2761 | GCTGAAACGG | GGGAAATAAA | ATGACAAACA | AAGAAAAAGA | GTTATTTGCT | GAAAATGAGG |
| 2821 | AATTAAAAAA | AGAAATTAAG | GACTTAAAAG | AGCGTATTGA | AAGATACAGA | GAAATGGAAG |
| 2881 | TTGAATTAAG | TACAACAATA | GATTTATTGA | GAGGAGGGAT | TATTGAATAA | ATAAAAGCCC |
| 2941 | CCCTGACGAA | AGTCGACGGC | AATAGTTACC | CTTATTATCA | AGATAAGAAA | GAAAAGGATT |
| 3001 | TTTCGCTACG | CTCAAATCCT | TTAAAAAAAC | ACAAAAGAGC | ACATTTTTTA | ATGTGGTCTT |
| 3061 | TATTCTTCAA | CTAAAGCACC | CATTAGTTCA | ACAAACGAAA | ATTGGATAAA | GTGGGATATT |
| 3121 | TTTAAAATAT | ATATTTATGT | TACAGTAATA | TTGACTTTTA | AAAAAGGATT | GATTCTAATG |
| 3181 | AAGAAAGCAG | ACAAGTAAGC | CTCCTAAATT | CACTTTAGAT | AAAAATTTAG | GAGGCATATC |
| 3241 | AAATGAACTT | TAATAAAATT | GATTTAGACA | ATTGGAAGAG | AAAAGAGATA | TTTAATCATT |
| 3301 | ATTTGAACCA | ACAAACGACT | TTTAGTATAA | CCACAGAAAT | TGATATTAGT | GTTTTATACC |
| 3361 | GAAACATAAA | ACAAGAAGGA | TATAAATTTT | ACCCTGCATT | TATTTTCTTA | GTGACAAGGG |
| 3421 | TGATAAACTC | AAATACAGCT | TTTAGAACTG | GTTACAATAG | CGACGGAGAG | TTAGGTTATT |
| 3481 | GGGATAAGTT | AGAGCCACTT | TATACAATTT | TTGATGGTGT | ATCTAAAACA | TTCTCTGGTA |
| 3541 | TTTGGACTCC | TGTAAAGAAT | GACTTCAAAG | AGTTTTATGA | TTTATACCTT | TCTGATGTAG |
| 3601 | AGAAATATAA | TGGTTCGGGG | AAATTGTTTC | CCAAAACACC | TATACCTGAA | AATGCTTTTT |
| 3661 | CTCTTTCTAT | TATTCCTTGG | ACTTCATTTA | CTGGGTTTAA | CTTAAATATC | AATAATAATA |

3721 GTAATTACCT TCTACCCATT ATTACAGCAG GAAAATTCAT TAATAAAGGT AATTCAATAT

3781 ATTTACCGCT ATCTTTACAG GTACATCATT CTGTTTGTGA TGGTTATCAT GCAGGATTGT

3841 TTATGAACTC TATTCAGGAA TTGTCAGATA GGCCTAATGA CTGGCTTTTA TAATATGAGA

3901 TAATGCCGAC TGTACTTTTT ACAGTCGGTT TTCTAATGTC ACTAACCTGC CCCGTTAGTT

3961 GAAGAAGGTT TTTATATTAC AGCTCCA

**pNZ-pPepN-USP-TATk-GFP (Amino Acid sequence; SEQ ID No 19)** 
MAKKKIISAILMSTVILSAAAPLSGVYADAAQPARRARRTKLAAYARKAARQARAPVATVSKGEELFTGVVPILVEL
DGDVNGHKFSVSGEGEGDATYGKLTLKFICTTGKLPVPWPTLVTTLTYGVQCFSRYPDHMKQHDFFKSAMPEGYVQE
RTIFFKDDGNYKTRAEVKFEGDTLVNRIELKGIDFKEDGNILGHKLEYNYNSHNVYIMADKQKNGIKVNFKIRHNIE
DGSVQLADHYQQNTPIGDGPVLLPDNHYLSTQSALSKDPNEKRDHMVLLEFVTAAGITLGMDELYKSGLRSRGGPEQ
KLISEEDLN-
 **pNZ-pPepN USP-GFP (DNA sequence; SEQ ID No 20)**

| Name | location |
|---|---|
| pPepN | 1..130 |
| Usp45 | 131..211 |
| GFP | 212..925 |
| Myc tag | 962..985 |
| Factor Xa site | rev:1371..1382 |
| repA | 1993..2691 |
| cat | 3150..3800 |

1 CTCTGTAAAA GCTGTCAGTA GACAGTTTTT TTAATAAGTT AAAGAAAAGA TGTAATTTTT
61 CTTTGTACTC GAAATTTTCT ATTCAATTTG ATATAATTAT ATTAATACTG AATATTTAGG
121 AGAAGATATG GCTAAAAAAA AGATTATCTC AGCTATTTTA ATGTCTACAG TGATACTTTC
181 TGCTGCAGCC CCGTTGTCAG GTGTTTACGC TGTGAGCAAG GGCGAGGAGC TGTTCACCGG
241 GGTGGTGCCC ATCCTGGTCG AGCTGGACGG CGACGTAAAC GGCCACAAGT TCAGCGTGTC
301 CGGCGAGGGC GAGGGCGATG CCACCTACGG CAAGCTGACC CTGAAGTTCA TCTGCACCAC
361 CGGCAAGCTG CCCGTGCCCT GGCCCACCCT CGTGACCACC CTGACCTACG GCGTGCAGTG
421 CTTCAGCCGC TACCCCGACC ACATGAAGCA GCACGACTTC TTCAAGTCCG CCATGCCCGA
481 AGGCTACGTC CAGGAGCGCA CCATCTTCTT CAAGGACGAC GGCAACTACA AGACCCGCGC
541 CGAGGTGAAG TTCGAGGGCG ACACCCTGGT GAACCGCATC GAGCTGAAGG GCATCGACTT
601 CAAGGAGGAC GGCAACATCC TGGGGCACAA GCTGGAGTAC AACTACAACA GCCACAACGT
661 CTATATCATG GCCGACAAGC AGAAGAACGG CATCAAGGTG AACTTCAAGA TCCGCCACAA
721 CATCGAGGAC GGCAGCGTGC AGCTCGCCGA CCACTACCAG CAGAACACCC CCATCGGCGA
781 CGGCCCCGTG CTGCTGCCCG ACAACCACTA CCTGAGCACC CAGTCCGCCC TGAGCAAAGA

841 CCCCAACGAG AAGCGCGATC ACATGGTCCT GCTGGAGTTC GTGACCGCCG CCGGGATCAC
901 TCTCGGCATG GACGAGCTGT ACAAGTCCGG ACTCAGATCT CGAGGAGGGC CCGAACAAAA
961 ACTCATCTCA GAAGAGGATC TGAATTGAAC CAAAATTAGA AAACCAAGGC TTGAAACGTT
1021 CAATTGAAAT GGCAATTAAA CAAATTACAG CACGTGTTGC TTTGATTGAT AGCCAAAAAG
1081 CAGCAGTTGA TAAAGCAATT ACTGATATTG CTGAAAAATT GTAATTTATA AATAAAAATC
1141 ACCTTTTAGA GGTGGTTTTT TTATTTATAA ATTATTCGTT TGATTTCGCT TTCGATAGAA
1201 CAATCAAATC GTTTCTGAGA CGTTTTAGCG TTTATTTCGT TTAGTTATCG GCATAATCGT
1261 TAAAACAGGC GTTATCGTAG CGTAAAAGCC CTTGAGCGTA GCGTGCTTTG CAGCGAAGAT
1321 GTTGTCTGTT AGATTATGAA AGCCGATGAC TGAATGAAAT AATAAGCGCA GCGTCCTTCT
1381 ATTTCGGTTG GAGGAGGCTC AAGGGAGTTT GAGGGAATGA AATTCCCTCA TGGGTTTGAT
1441 TTTAAAAATT GCTTGCAATT TTGCCGAGCG GTAGCGCTGG AAAATTTTTG AAAAAAATTT
1501 GGAATTTGGA AAAAAATGGG GGGAAAGGAA GCGAATTTTG CTTCCGTACT ACGACCCCCC
1561 ATTAAGTGCC GAGTGCCAAT TTTTGTGCCA AAAACGCTCT ATCCCAACTG GCTCAAGGGT
1621 TTGAGGGGTT TTTCAATCGC CAACGAATCG CCAACGTTTT CGCCAACGTT TTTTATAAAT
1681 CTATATTTAA GTAGCTTTAT TGTTGTTTTT ATGATTACAA AGTGATACAC TAATTTTATA
1741 AAATTATTTG ATTGGAGTTT TTTAAATGGT GATTTCAGAA TCGAAAAAAA GAGTTATGAT
1801 TTCTCTGACA AAAGAGCAAG ATAAAAAATT AACAGATATG GCGAAACAAA AAGGTTTTTC
1861 AAAATCTGCG GTTGCGGCGT TAGCTATAGA AGAATATGCA AGAAAGGAAT CAGAACAAAA
1921 AAAATAAGCG AAAGCTCGCG TTTTTAGAAG GATACGAGTT TTCGCTACTT GTTTTTGATA
1981 AGGTAATATA TCATGGCTAT TAAAAATACT AAAGCTAGAA ATTTTGGATT TTTATTATAT
2041 CCTGACTCAA TTCCTAATGA TTGGAAAGAA AAATTAGAGA GTTTGGGCGT ATCTATGGCT
2101 GTCAGTCCTT TACACGATAT GGACGAAAAA AAAGATAAAG ATACATGGAA TAGTAGTGAT
2161 GTTATACGAA ATGGAAAGCA CTATAAAAAA CCACACTATC ACGTTATATA TATTGCACGA
2221 AATCCTGTAA CAATAGAAAG CGTTAGGAAC AAGATTAAGC GAAAATTGGG GAATAGTTCA
2281 GTTGCTCATG TTGAGATACT TGATTATATC AAAGGTTCAT ATGAATATTT GACTCATGAA
2341 TCAAAGGACG CTATTGCTAA GAATAAACAT ATATACGACA AAAAAGATAT TTTGAACATT
2401 AATGATTTTG ATATTGACCG CTATATAACA CTTGATGAAA GCCAAAAAAG AGAATTGAAG
2461 AATTTACTTT TAGATATAGT GGATGACTAT AATTTGGTAA ATACAAAAGA TTTAATGGCT
2521 TTTATTCGCC TTAGGGGAGC GGAGTTTGGA ATTTTAAATA CGAATGATGT AAAAGATATT
2581 GTTTCAACAA ACTCTAGCGC CTTTAGATTA TGGTTTGAGG GCAATTATCA GTGTGGATAT
2641 AGAGCAAGTT ATGCAAAGGT TCTTGATGCT GAAACGGGGG AAATAAAATG ACAAACAAAG
2701 AAAAAGAGTT ATTTGCTGAA AATGAGGAAT TAAAAAAAGA AATTAAGGAC TTAAAAGAGC
2761 GTATTGAAAG ATACAGAGAA ATGGAAGTTG AATTAAGTAC AACAATAGAT TTATTGAGAG
2821 GAGGGATTAT TGAATAAATA AAAGCCCCCC TGACGAAAGT CGACGGCAAT AGTTACCCTT
2881 ATTATCAAGA TAAGAAAGAA AAGGATTTTT CGCTACGCTC AAATCCTTTA AAAAAACACA
2941 AAAGACCACA TTTTTTAATG TGGTCTTTAT TCTTCAACTA AAGCACCCAT TAGTTCAACA
3001 AACGAAAATT GGATAAAGTG GGATATTTTT AAAATATATA TTTATGTTAC AGTAATATTG
3061 ACTTTTAAAA AAGGATTGAT TCTAATGAAG AAAGCAGACA AGTAAGCCTC CTAAATTCAC
3121 TTTAGATAAA AATTTAGGAG GCATATCAAA TGAACTTTAA TAAAATTGAT TTAGACAATT
3181 GGAAGAGAAA AGAGATATTT AATCATTATT TGAACCAACA AACGACTTTT AGTATAACCA
3241 CAGAAATTGA TATTAGTGTT TTATACCGAA ACATAAAACA AGAAGGATAT AAATTTTACC
3301 CTGCATTTAT TTTCTTAGTG ACAAGGGTGA TAAACTCAAA TACAGCTTTT AGAACTGGTT

3361 ACAATAGCGA CGGAGAGTTA GGTTATTGGG ATAAGTTAGA GCCACTTTAT ACAATTTTTG
3421 ATGGTGTATC TAAAACATTC TCTGGTATTT GGACTCCTGT AAAGAATGAC TTCAAAGAGT
3481 TTTATGATTT ATACCTTTCT GATGTAGAGA AATATAATGG TTCGGGGAAA TTGTTTCCCA
3541 AAACACCTAT ACCTGAAAAT GCTTTTTCTC TTTCTATTAT TCCTTGGACT TCATTTACTG
3601 GGTTTAACTT AAATATCAAT AATAATAGTA ATTACCTTCT ACCCATTATT ACAGCAGGAA
3661 AATTCATTAA TAAAGGTAAT TCAATATATT TACCGCTATC TTTACAGGTA CATCATTCTG
3721 TTTGTGATGG TTATCATGCA GGATTGTTTA TGAACTCTAT TCAGGAATTG TCAGATAGGC
3781 CTAATGACTG GCTTTTATAA TATGAGATAA TGCCGACTGT ACTTTTTACA GTCGGTTTTC
3841 TAATGTCACT AACCTGCCCC GTTAGTTGAA GAAGGTTTTT ATATTACAGC TCCA

**pNZ-pPepN USP-GFP (Amino Acid sequence; SEQ ID No 21)** 
MAKKKIISAILMSTVILSAAAPLSGVYAVSKGEELFTGVVPILVELDGDVNGHKFSVSGEGEGDATYGKLTLKFICT
TGKLPVPWPTLVTTLTYGVQCFSRYPDHMKQHDFFKSAMPEGYVQERTIFFKDDGNYKTRAEVKFEGDTLVNRIELK
GIDFKEDGNILGHKLEYNYNSHNVYIMADKQKNGIKVNFKIRHNIEDGSVQLADHYQQNTPIGDGPVLLPDNHYLST
QSALSKDPNEKRDHMVLLEFVTAAGITLGMDELYKSGLRSRGGPEQKLISEEDLN-
 **pNZ-pNisA-TATk-CDKL5-FLAG (DNA sequence; SEQ ID No 22)**

| Name | | location |
|---|---|---|
| | pNisA | 5..188 |
| | TATk-CDKL5 | 231..3341 |
| | repC | 3888..4097 |
| | repA | 4365..5063 |
| | CAT | 5522..6172 |

1 AGATCTAGTC TTATAACTAT ACTGACAATA GAAACATTAA CAAATCTAAA ACAGTCTTAA
61 TTCTATCTTG AGAAAGTATT GGTAATAATA TTATTGTCGA TAACGCGAGC ATAATAAACG
121 GCTCTGATTA AATTCTGAAG TTTGTTAGAT ACAATGATTT CGTTCGAAGG AACTACAAAA
181 TAAATTATAA GGAGGCACTC ACCATGGGTA CTGCAGGCAT GCGGTACCTT ATGGAGACAG
241 ACACACTCCT GCTATGGGTA CTGCTGCTCT GGGTTCCAGG TTCCACTGGT GACGCGGCCC
301 AGCCGGCCAG GCGCGCGCGC CGTACGAAGC TTGCGGCCTA CGCCAGAAAG GCCGCCAGGC
361 AGGCCAGGGC ACCGGTGAAG ATTCCTAACA TTGGTAATGT GATGAATAAA TTTGAGATCC
421 TTGGGGTTGT AGGTGAAGGA GCCTATGGAG TTGTACTTAA ATGCAGACAC AAGGAAACAC
481 ATGAAATTGT GGCGATCAAG AAATTCAAGG ACAGTGAAGA AAATGAAGAA GTCAAAGAAA
541 CGACTTTACG AGAGCTTAAA ATGCTTCGGA CTCTCAAGCA GGAAAACATT GTGGAGTTGA
601 AGGAAGCATT TCGTCGGAGG GGAAAGTTGT ACTTGGTGTT TGAGTATGTT GAAAAAAATA
661 TGCTCGAATT GCTGGAAGAA ATGCCAAATG GAGTTCCACC TGAGAAAGTA AAAAGCTACA
721 TCTATCAGCT AATCAAGGCT ATTCACTGGT GCCATAAGAA TGATATTGTC CATCGAGATA
781 TAAAACCAGA AAATCTCTTA ATCAGCCACA ATGATGTCCT AAAACTGTGT GACTTTGGTT
841 TTGCTCGTAA TCTGTCAGAA GGCAATAATG CTAATTACAC AGAGTACGTT GCCACCAGAT
901 GGTATCGGTC CCCAGAACTC TTACTTGGCG CTCCCTATGG AAAGTCCGTG GACATGTGGT

961 CGGTGGGCTG TATTCTTGGG GAGCTTAGCG ATGGACAGCC TTTATTTCCT GGAGAAAGTG
1021 AAATTGACCA ACTTTTTACT ATTCAGAAGG TGCTAGGACC ACTTCCATCT GAGCAGATGA
1081 AGCTTTTCTA CAGTAATCCT CGCTTCCATG GGCTCCGGTT TCCAGCTGTT AACCATCCTC
1141 AGTCCTTGGA AAGAAGATAC CTTGGAATTT TGAATAGTGT TCTACTTGAC CTAATGAAGA
1201 ATTTACTGAA GTTGGACCCA GCTGACAGAT ACTTGACAGA ACAGTGTTTG AATCACCCTA
1261 CATTTCAAAC CCAGAGACTT CTGGATCGTT CTCCTTCAAG GTCAGCAAAA AGAAAACCTT
1321 ACCATGTGGA AAGCAGCACA TTGTCTAATA GAAACCAAGC CGGCAAAAGC ACTGCTTTGC
1381 AGTCTCACCA CAGATCTAAC AGCAAGGACA TCCAGAACCT GAGTGTAGGC CTGCCCCGGG
1441 CTGACGAAGG TCTCCCTGCC AATGAAAGCT TCCTAAATGG AAACCTTGCT GGAGCTAGTC
1501 TTAGTCCACT GCACACCAAA ACCTACCAAG CAAGCAGCCA GCCTGGGTCT ACCAGCAAAG
1561 ATCTCACCAA CAACAACATA CCACACCTTC TTAGCCCAAA AGAAGCCAAG TCAAAAACAG
1621 AGTTTGATTT TAATATTGAC CCAAAGCCTT CAGAAGGCCC AGGGACAAAG TACCTCAAGT
1681 CAAACAGCAG ATCTCAGCAG AACCGCCACT CATTCATGGA AAGCTCTCAA AGCAAAGCTG
1741 GGACACTGCA GCCCAATGAA AAGCAGAGTC GGCATAGCTA TATTGACACA ATTCCCCAGT
1801 CCTCTAGGAG TCCCTCCTAC AGGACCAAGG CCAAAAGCCA TGGGGCACTG AGTGACTCCA
1861 AGTCTGTGAG CAACCTTTCT GAAGCCAGGG CCCAAATTGC GGAGCCCAGT ACCAGTAGGT
1921 ACTTCCCATC TAGCTGCTTA GACTTGAATT CTCCCACCAG CCCAACCCCC ACCAGACACA
1981 GTGACACGAG AACTTTGCTC AGCCCTTCTG GAAGAAATAA CCGAAATGAG GGAACGCTGG
2041 ACTCACGTCG AACCACAACC AGACATTCTA AGACGATGGA GGAATTGAAG CTGCCGGAGC
2101 ACATGGACAG TAGCCATTCC CATTCACTGT CTGCACCTCA CGAATCTTTT TCTTATGGAC
2161 TGGGCTACAC CAGCCCCTTT TCTTCCCAGC AACGTCCTCA TAGGCATTCT ATGTATGTGA
2221 CCCGTGACAA AGTGAGAGCC AAGGGCTTGG ATGGAAGCTT GAGCATAGGG CAAGGGATGG
2281 CAGCTAGAGC CAACAGCCTG CAACTCTTGT CACCCCAGCC TGGAGAACAG CTCCCTCCAG
2341 AGATGACTGT GGCAAGATCT TCGGTCAAAG AGACCTCCAG AGAAGGCACC TCTTCCTTCC
2401 ATACACGCCA GAAGTCTGAG GGTGGAGTGT ATCATGACCC ACACTCTGAT GATGGCACAG
2461 CCCCCAAAGA AAATAGACAC CTATACAATG ATCCTGTGCC AAGGAGAGTT GGTAGCTTTT
2521 ACAGAGTGCC ATCTCCACGT CCAGACAATT CTTTCCATGA AAATAATGTG TCAACTAGAG
2581 TTTCTTCTCT ACCATCAGAG AGCAGTTCTG GAACCAACCA CTCAAAAAGA CAACCAGCAT
2641 TCGATCCATG GAAAAGTCCT GAAAATATTA GTCATTCGGA GCAACTCAAG GAAAAAGAGA
2701 AGCAAGGATT TTTCAGGTCA ATGAAAAAGA AAAAGAAGAA ATCTCAAACA GTACCCAATT
2761 CCGACAGCCC TGATCTTCTG ACGTTGCAGA AATCCATTCA TTCTGCTAGC ACTCCAAGCA
2821 GCAGACCAAA GGAGTGGCGC CCCGAGAAGA TCTCAGATCT GCAGACCCAA AGCCAGCCAT
2881 TAAAGTCACT GCGCAAGTTG TTACATCTCT CTTCGGCCTC AAATCACCCG GCTTCCTCAG
2941 ATCCCCGCTT CCAGCCCTTA ACAGCTCAAC AAACCAAAAA TTCCTTCTCA GAAATTCGGA
3001 TTCACCCCCT GAGCCAGGCC TCTGGCGGGA GCAGCAACAT CCGGCAGGAA CCCGCACCGA
3061 AGGGCAGGCC AGCCCTCCAG CTGCCAGGTC AGATGGATCC TGGTTGGCAT GTGTCCTCTG
3121 TGACCAGGAG TGCCACAGAG GGCCCTTCCT ACTCTGAACA GCTGGGTGCC AAAAGTGGGC
3181 CAAATGGGCA CCCCTATAAC AGAACAAATC GCTCACGAAT GCCAAATCTG AATGATTTAA
3241 AAGAGACAGC CTTGTCTAGA GGATCCCGGG CTGACTACAA AGACCATGAC GGTGATTATA
3301 AAGATCATGA CATCGACTAC AAGGATGACG ATGACAAGTA GGAGCTCAAG CTTTCTTTGA
3361 ACCAAAATTA GAAAACCAAG GCTTGAAACG TTCAATTGAA ATGGCAATTA AACAAATTAC
3421 AGCACGTGTT GCTTTGATTG ATAGCCAAAA AGCAGCAGTT GATAAAGCAA TTACTGATAT

3481 TGCTGAAAAA TTGTAATTTA TAAATAAAAA TCACCTTTTA GAGGTGGTTT TTTTATTTAT
3541 AAATTATTCG TTTGATTTCG CTTTCGATAG AACAATCAAA TCGTTTCTGA GACGTTTTAG
3601 CGTTTATTTC GTTTAGTTAT CGGCATAATC GTTAAAACAG GCGTTATCGT AGCGTAAAAG
3661 CCCTTGAGCG TAGCGTGCTT TGCAGCGAAG ATGTTGTCTG TTAGATTATG AAAGCCGATG
3721 ACTGAATGAA ATAATAAGCG CAGCGTCCTT CTATTTCGGT TGGAGGAGGC TCAAGGGAGT
3781 TTGAGGGAAT GAAATTCCCT CATGGGTTTG ATTTTAAAAA TTGCTTGCAA TTTTGCCGAG
3841 CGGTAGCGCT GGAAAATTTT TGAAAAAAAT TTGGAATTTG GAAAAAAATG GGGGGAAAGG
3901 AAGCGAATTT TGCTTCCGTA CTACGACCCC CCATTAAGTG CCGAGTGCCA ATTTTTGTGC
3961 CAAAAACGCT CTATCCCAAC TGGCTCAAGG GTTTGAGGGG TTTTTCAATC GCCAACGAAT
4021 CGCCAACGTT TTCGCCAACG TTTTTTATAA ATCTATATTT AAGTAGCTTT ATTGTTGTTT
4081 TTATGATTAC AAAGTGATAC ACTAATTTTA TAAAATTATT TGATTGGAGT TTTTTAAATG
4141 GTGATTTCAG AATCGAAAAA AAGAGTTATG ATTTCTCTGA CAAAAGAGCA AGATAAAAAA
4201 TTAACAGATA TGGCGAAACA AAAAGGTTTT TCAAAATCTG CGGTTGCGGC GTTAGCTATA
4261 GAAGAATATG CAAGAAAGGA ATCAGAACAA AAAAAATAAG CGAAAGCTCG CGTTTTTAGA
4321 AGGATACGAG TTTTCGCTAC TTGTTTTTGA TAAGGTAATA TATCATGGCT ATTAAAAATA
4381 CTAAAGCTAG AAATTTTGGA TTTTTATTAT ATCCTGACTC AATTCCTAAT GATTGGAAAG
4441 AAAAATTAGA GAGTTTGGGC GTATCTATGG CTGTCAGTCC TTTACACGAT ATGGACGAAA
4501 AAAAAGATAA AGATACATGG AATAGTAGTG ATGTTATACG AAATGGAAAG CACTATAAAA
4561 AACCACACTA TCACGTTATA TATATTGCAC GAAATCCTGT AACAATAGAA AGCGTTAGGA
4621 ACAAGATTAA GCGAAAATTG GGGAATAGTT CAGTTGCTCA TGTTGAGATA CTTGATTATA
4681 TCAAAGGTTC ATATGAATAT TTGACTCATG AATCAAAGGA CGCTATTGCT AAGAATAAAC
4741 ATATATACGA CAAAAAAGAT ATTTTGAACA TTAATGATTT TGATATTGAC CGCTATATAA
4801 CACTTGATGA AAGCCAAAAA AGAGAATTGA AGAATTTACT TTTAGATATA GTGGATGACT
4861 ATAATTTGGT AAATACAAAA GATTTAATGG CTTTTATTCG CCTTAGGGGA GCGGAGTTTG
4921 GAATTTTAAA TACGAATGAT GTAAAAGATA TTGTTTCAAC AAACTCTAGC GCCTTTAGAT
4981 TATGGTTTGA GGGCAATTAT CAGTGTGGAT ATAGAGCAAG TTATGCAAAG GTTCTTGATG
5041 CTGAAACGGG GGAAATAAAA TGACAAACAA AGAAAAAGAG TTATTTGCTG AAAATGAGGA
5101 ATTAAAAAAA GAAATTAAGG ACTTAAAAGA GCGTATTGAA AGATACAGAG AAATGGAAGT
5161 TGAATTAAGT ACAACAATAG ATTTATTGAG AGGAGGGATT ATTGAATAAA TAAAAGCCCC
5221 CCTGACGAAA GTCGACGGCA ATAGTTACCC TTATTATCAA GATAAGAAAG AAAAGGATTT
5281 TTCGCTACGC TCAAATCCTT TAAAAAAACA CAAAAGACCA CATTTTTTAA TGTGGTCTTT
5341 ATTCTTCAAC TAAAGCACCC ATTAGTTCAA CAAACGAAAA TTGGATAAAG TGGGATATTT
5401 TTAAAATATA TATTTATGTT ACAGTAATAT TGACTTTTAA AAAAGGATTG ATTCTAATGA
5461 AGAAAGCAGA CAAGTAAGCC TCCTAAATTC ACTTTAGATA AAAATTTAGG AGGCATATCA
5521 AATGAACTTT AATAAAATTG ATTTAGACAA TTGGAAGAGA AAAGAGATAT TTAATCATTA
5581 TTTGAACCAA CAAACGACTT TTAGTATAAC CACAGAAATT GATATTAGTG TTTTATACCG
5641 AAACATAAAA CAAGAAGGAT ATAAATTTTA CCCTGCATTT ATTTTCTTAG TGACAAGGGT
5701 GATAAACTCA AATACAGCTT TTAGAACTGG TTACAATAGC GACGGAGAGT TAGGTTATTG
5761 GGATAAGTTA GAGCCACTTT ATACAATTTT TGATGGTGTA TCTAAAACAT TCTCTGGTAT
5821 TTGGACTCCT GTAAAGAATG ACTTCAAAGA GTTTTATGAT TTATACCTTT CTGATGTAGA
5881 GAAATATAAT GGTTCGGGGA AATTGTTTCC CAAAACACCT ATACCTGAAA ATGCTTTTTC
5941 TCTTTCTATT ATTCCTTGGA CTTCATTTAC TGGGTTTAAC TTAAATATCA ATAATAATAG

6001 TAATTACCTT CTACCCATTA TTACAGCAGG AAAATTCATT AATAAAGGTA ATTCAATATA
6061 TTTACCGCTA TCTTTACAGG TACATCATTC TGTTTGTGAT GGTTATCATG CAGGATTGTT
6121 TATGAACTCT ATTCAGGAAT TGTCAGATAG GCCTAATGAC TGGCTTTTAT AATATGAGAT
6181 AATGCCGACT GTACTTTTTA CAGTCGGTTT TCTAATGTCA CTAACCTGCC CCGTTAGTTG
6241 AAGAAGGTTT TTATATTACA GCTCCA

**pNZ-pNisA-TATk-CDKL5-FLAG (Amino Acid sequence; SEQ ID No 23)** 
METDTLLLWVLLLWVPGSTGDAAQPARRARRTKLAAYARKAARQARAPVKIPNIGNVMNKFEILGVVGEGAYGVVLK
CRHKETHEIVAIKKFKDSEENEEVKETTLRELKMLRTLKQENIVELKEAFRRRGKLYLVFEYVEKNMLELLEEMPNG
VPPEKVKSYIYQLIKAIHWCHKNDIVHRDIKPENLLISHNDVLKLCDFGFARNLSEGNNANYTEYVATRWYRSPELL
LGAPYGKSVDMWSVGCILGELSDGQPLFPGESEIDQLFTIQKVLGPLPSEQMKLFYSNPRFHGLRFPAVNHPQSLER
RYLGILNSVLLDLMKNLLKLDPADRYLTEQCLNHPTFQTQRLLDRSPSRSAKRKPYHVESSTLSNRNQAGKSTALQS
HHRSNSKDIQNLSVGLPRADEGLPANESFLNGNLAGASLSPLHTKTYQASSQPGSTSKDLTNNNIPHLLSPKEAKSK
TEFDFNIDPKPSEGPGTKYLKSNSRSQQNRHSFMESSQSKAGTLQPNEKQSRHSYIDTIPQSSRSPSYRTKAKSHGA
LSDSKSVSNLSEARAQIAEPSTSRYFPSSCLDLNSPTSPTPTRHSDTRTLLSPSGRNNRNEGTLDSRRTTTRHSKTM
EELKLPEHMDSSHSHSLSAPHESFSYGLGYTSPFSSQQRPHRHSMYVTRDKVRAKGLDGSLSIGQGMAARANSLQLL
SPQPGEQLPPEMTVARSSVKETSREGTSSFHTRQKSEGGVYHDPHSDDGTAPKENRHLYNDPVPRRVGSFYRVPSPR
PDNSFHENNVSTRVSSLPSESSSGTNHSKRQPAFDPWKSPENISHSEQLKEKEKQGFFRSMKKKKKSQTVPNSDSP
DLLTLQKSIHSASTPSSRPKEWRPEKISDLQTQSQPLKSLRKLLHLSSASNHPASSDPRFQPLTAQQTKNSFSEIRI
HPLSQASGGSSNIRQEPAPKGRPALQLPGQMDPGWHVSSVTRSATEGPSYSEQLGAKSGPNGHPYNRTNRSRMPNLN
DLKETALSRGSRADYKDHDGDYKDHDIDYKDDDDK-
 **pNZ-pNisA-USP-TATk-CDKL5-FLAG (DNA sequence; SEQ ID No 24)**

| Name | | location |
|---|---|---|
| | promoter | 5..188 |
| | Usp45 | 231..314 |
| | TATk-CDKL5 | 315..3425 |
| | Terminator | 3578..3630 |
| | repC | 3972..4181 |
| | repA | 4449..5147 |
| | CAT | 5606..6256 |

1 AGATCTAGTC TTATAACTAT ACTGACAATA GAAACATTAA CAAATCTAAA ACAGTCTTAA
61 TTCTATCTTG AGAAAGTATT GGTAATAATA TTATTGTCGA TAACGCGAGC ATAATAAACG
121 GCTCTGATTA AATTCTGAAG TTTGTTAGAT ACAATGATTT CGTTCGAAGG AACTACAAAA
181 TAAATTATAA GGAGGCACTC ACCATGGGTA CTGCAGGCAT GCGGTACCtt ATGGCTAAAA
241 AAAAGATTAT CTCAGCTATT TTAATGTCTA CAGTGATACT TTCTGCTGCA GCCCCGTTGT
301 CAGGTGTTTA CGCTatggag acagacacac tcctgctatg ggtactgctg ctctgggttc
361 caggttccac tggtgacgcg gcccagccgg ccaggcgcgc gcgccgtacg aagcttgcgg
421 cctacgccag aaaggccgcc aggcaggcca gggcaccggt gaagattcct aacattggta

481 atgtgatgaa taaatttgag atccttgggg ttgtaggtga aggagcctat ggagttgtac
541 ttaaatgcag acacaaggaa acacatgaaa ttgtggcgat caagaaattc aaggacagtg
601 aagaaaatga agaagtcaaa gaaacgactt tacgagagct taaaatgctt cggactctca
661 agcaggaaaa cattgtggag ttgaaggaag catttcgtcg gaggggaaag ttgtacttgg
721 tgtttgagta tgttgaaaaa aatatgctcg aattgctgga agaaatgcca aatggagttc
781 cacctgagaa agtaaaaagc tacatctatc agctaatcaa ggctattcac tggtgccata
841 agaatgatat tgtccatcga gatataaaac cagaaaatct cttaatcagc cacaatgatg
901 tcctaaaact gtgtgacttt ggttttgctc gtaatctgtc agaaggcaat aatgctaatt
961 acacagagta cgttgccacc agatggtatc ggtccccaga actcttactt ggcgctccct
1021 atggaaagtc cgtggacatg tggtcggtgg gctgtattct tggggagctt agcgatggac
1081 agcctttatt tcctggagaa agtgaaattg accaactttt tactattcag aaggtgctag
1141 gaccacttcc atctgagcag atgaagcttt tctacagtaa tcctcgcttc catgggctcc
1201 ggtttccagc tgttaaccat cctcagtcct tggaaagaag ataccttgga attttgaata
1261 gtgttctact tgacctaatg aagaatttac tgaagttgga cccagctgac agatacttga
1321 cagaacagtg tttgaatcac cctacatttc aaacccagag acttctggat cgttctcctt
1381 caaggtcagc aaaaagaaaa ccttaccatg tggaaagcag cacattgtct aatagaaacc
1441 aagccggcaa aagcactgct ttgcagtctc accacagatc taacagcaag gacatccaga
1501 acctgagtgt aggcctgccc cgggctgacg aaggtctccc tgccaatgaa agcttcctaa
1561 atggaaacct tgctggagct agtcttagtc cactgcacac caaaacctac caagcaagca
1621 gccagcctgg gtctaccagc aaagatctca ccaacaacaa cataccacac cttcttagcc
1681 caaaagaagc caagtcaaaa acagagtttg attttaatat tgacccaaag ccttcagaag
1741 gcccagggac aaagtacctc aagtcaaaca gcagatctca gcagaaccgc cactcattca
1801 tggaaagctc tcaaagcaaa gctgggacac tgcagcccaa tgaaaagcag agtcggcata
1861 gctatattga cacaattccc cagtcctcta ggagtccctc ctacaggacc aaggccaaaa
1921 gccatggggc actgagtgac tccaagtctg tgagcaacct ttctgaagcc agggcccaaa
1981 ttgcggagcc cagtaccagt aggtacttcc catctagctg cttagacttg aattctccca
2041 ccagcccaac ccccaccaga cacagtgaca cgagaacttt gctcagccct tctggaagaa
2101 ataaccgaaa tgagggaacg ctggactcac gtcgaaccac aaccagacat tctaagacga
2161 tggaggaatt gaagctgccg gagcacatgg acagtagcca ttcccattca ctgtctgcac
2221 ctcacgaatc tttttcttat ggactgggct acaccagccc cttttcttcc cagcaacgtc
2281 ctcataggca ttctatgtat gtgacccgtg acaaagtgag agccaagggc ttggatggaa
2341 gcttgagcat agggcaaggg atggcagcta gagccaacag cctgcaactc ttgtcacccc
2401 agcctggaga acagctccct ccagagatga ctgtggcaag atcttcggtc aaagagacct
2461 ccagagaagg cacctcttcc ttccatacac gccagaagtc tgagggtgga gtgtatcatg
2521 acccacactc tgatgatggc acagccccca aagaaaatag acacctatac aatgatcctg
2581 tgccaaggag agttggtagc ttttacagag tgccatctcc acgtccagac aattctttcc
2641 atgaaaataa tgtgtcaact agagtttctt ctctaccatc agagagcagt tctggaacca
2701 accactcaaa aagacaacca gcattcgatc catggaaaag tcctgaaaat attagtcatt
2761 cggagcaact caaggaaaaa gagaagcaag gatttttcag gtcaatgaaa aagaaaaaga
2821 agaaatctca aacagtaccc aattccgaca gccctgatct tctgacgttg cagaaatcca
2881 ttcattctgc tagcactcca agcagcagac caaaggagtg gcgccccgag aagatctcag
2941 atctgcagac ccaaagccag ccattaaagt cactgcgcaa gttgttacat ctctcttcgg

3001 cctcaaatca cccggcttcc tcagatcccc gcttccagcc cttaacagct caacaaacca
3061 aaaattcctt ctcagaaatt cggattcacc ccctgagcca ggcctctggc gggagcagca
3121 acatccggca ggaacccgca ccgaagggca ggccagccct ccagctgcca ggtcagatgg
3181 atcctggttg gcatgtgtcc tctgtgacca ggagtgccac agagggccct tcctactctg
3241 aacagctggg tgccaaaagt gggccaaatg ggcaccccta taacagaaca aatcgctcac
3301 gaatgccaaa tctgaatgat ttaaaagaga cagccttgtc tagaggatcc cgggctgact
3361 acaaagacca tgacggtgat tataaagatc atgacatcga ctacaaggat gacgatgaca
3421 agtagGAGCT CAAGCTTTCT TTGAACCAAA ATTAGAAAAC CAAGGCTTGA AACGTTCAAT
3481 TGAAATGGCA ATTAAACAAA TTACAGCACG TGTTGCTTTG ATTGATAGCC AAAAAGCAGC
3541 AGTTGATAAA GCAATTACTG ATATTGCTGA AAAATTGTAA TTTATAAATA AAAATCACCT
3601 TTTAGAGGTG GTTTTTTTAT TTATAAATTA TTCGTTTGAT TTCGCTTTCG ATAGAACAAT
3661 CAAATCGTTT CTGAGACGTT TTAGCGTTTA TTTCGTTTAG TTATCGGCAT AATCGTTAAA
3721 ACAGGCGTTA TCGTAGCGTA AAAGCCCTTG AGCGTAGCGT GCTTTGCAGC GAAGATGTTG
3781 TCTGTTAGAT TATGAAAGCC GATGACTGAA TGAAATAATA AGCGCAGCGT CCTTCTATTT
3841 CGGTTGGAGG AGGCTCAAGG GAGTTTGAGG GAATGAAATT CCCTCATGGG TTTGATTTTA
3901 AAAATTGCTT GCAATTTTGC CGAGCGGTAG CGCTGGAAAA TTTTTGAAAA AAATTTGGAA
3961 TTTGGAAAAA AATGGGGGGA AAGGAAGCGA ATTTTGCTTC CGTACTACGA CCCCCCATTA
4021 AGTGCCGAGT GCCAATTTTT GTGCCAAAAA CGCTCTATCC CAACTGGCTC AAGGGTTTGA
4081 GGGGTTTTTC AATCGCCAAC GAATCGCCAA CGTTTTCGCC AACGTTTTTT ATAAATCTAT
4141 ATTTAAGTAG CTTTATTGTT GTTTTTATGA TTACAAAGTG ATACACTAAT TTTATAAAAT
4201 TATTTGATTG GAGTTTTTTA AATGGTGATT TCAGAATCGA AAAAAAGAGT TATGATTTCT
4261 CTGACAAAAG AGCAAGATAA AAAATTAACA GATATGGCGA AACAAAAAGG TTTTTCAAAA
4321 TCTGCGGTTG CGGCGTTAGC TATAGAAGAA TATGCAAGAA AGGAATCAGA ACAAAAAAAA
4381 TAAGCGAAAG CTCGCGTTTT TAGAAGGATA CGAGTTTTCG CTACTTGTTT TTGATAAGGT
4441 AATATATCAT GGCTATTAAA AATACTAAAG CTAGAAATTT TGGATTTTTA TTATATCCTG
4501 ACTCAATTCC TAATGATTGG AAAGAAAAAT TAGAGAGTTT GGGCGTATCT ATGGCTGTCA
4561 GTCCTTTACA CGATATGGAC GAAAAAAAAG ATAAAGATAC ATGGAATAGT AGTGATGTTA
4621 TACGAAATGG AAAGCACTAT AAAAAACCAC ACTATCACGT TATATATATT GCACGAAATC
4681 CTGTAACAAT AGAAAGCGTT AGGAACAAGA TTAAGCGAAA ATTGGGGAAT AGTTCAGTTG
4741 CTCATGTTGA GATACTTGAT TATATCAAAG GTTCATATGA ATATTTGACT CATGAATCAA
4801 AGGACGCTAT TGCTAAGAAT AAACATATAT ACGACAAAAA AGATATTTTG AACATTAATG
4861 ATTTTGATAT TGACCGCTAT ATAACACTTG ATGAAAGCCA AAAAAGAGAA TTGAAGAATT
4921 TACTTTTAGA TATAGTGGAT GACTATAATT TGGTAAATAC AAAAGATTTA ATGGCTTTTA
4981 TTCGCCTTAG GGGAGCGGAG TTTGGAATTT TAAATACGAA TGATGTAAAA GATATTGTTT
5041 CAACAAACTC TAGCGCCTTT AGATTATGGT TTGAGGGCAA TTATCAGTGT GGATATAGAG
5101 CAAGTTATGC AAAGGTTCTT GATGCTGAAA CGGGGGAAAT AAAATGACAA ACAAAGAAAA
5161 AGAGTTATTT GCTGAAAATG AGGAATTAAA AAAAGAAATT AAGGACTTAA AAGAGCGTAT
5221 TGAAAGATAC AGAGAAATGG AAGTTGAATT AAGTACAACA ATAGATTTAT TGAGAGGAGG
5281 GATTATTGAA TAAATAAAAG CCCCCCTGAC GAAAGTCGAC GGCAATAGTT ACCCTTATTA
5341 TCAAGATAAG AAAGAAAAGG ATTTTTCGCT ACGCTCAAAT CCTTTAAAAA AACACAAAAG
5401 ACCACATTTT TTAATGTGGT CTTTATTCTT CAACTAAAGC ACCCATTAGT TCAACAAACG
5461 AAAATTGGAT AAAGTGGGAT ATTTTTAAAA TATATATTTA TGTTACAGTA ATATTGACTT

5521 TTAAAAAAGG ATTGATTCTA ATGAAGAAAG CAGACAAGTA AGCCTCCTAA ATTCACTTTA
5581 GATAAAAATT TAGGAGGCAT ATCAAATGAA CTTTAATAAA ATTGATTTAG ACAATTGGAA
5641 GAGAAAAGAG ATATTTAATC ATTATTTGAA CCAACAAACG ACTTTTAGTA TAACCACAGA
5701 AATTGATATT AGTGTTTTAT ACCGAAACAT AAAACAAGAA GGATATAAAT TTTACCCTGC
5761 ATTTATTTTC TTAGTGACAA GGGTGATAAA CTCAAATACA GCTTTTAGAA CTGGTTACAA
5821 TAGCGACGGA GAGTTAGGTT ATTGGGATAA GTTAGAGCCA CTTTATACAA TTTTTGATGG
5881 TGTATCTAAA ACATTCTCTG GTATTTGGAC TCCTGTAAAG AATGACTTCA AAGAGTTTTA
5941 TGATTTATAC CTTTCTGATG TAGAGAAATA TAATGGTTCG GGGAAATTGT TTCCCAAAAC
6001 ACCTATACCT GAAAATGCTT TTTCTCTTTC TATTATTCCT TGGACTTCAT TTACTGGGTT
6061 TAACTTAAAT ATCAATAATA ATAGTAATTA CCTTCTACCC ATTATTACAG CAGGAAAATT
6121 CATTAATAAA GGTAATTCAA TATATTTACC GCTATCTTTA CAGGTACATC ATTCTGTTTG
6181 TGATGGTTAT CATGCAGGAT TGTTTATGAA CTCTATTCAG GAATTGTCAG ATAGGCCTAA
6241 TGACTGGCTT TTATAATATG AGATAATGCC GACTGTACTT TTTACAGTCG GTTTTCTAAT
6301 GTCACTAACC TGCCCCGTTA GTTGAAGAAG GTTTTTATAT TACAGCTCCA

**pNZ-pNisA-USP-TATk-CDKL5-FLAG (Amino Acid sequence; SEQ ID No 25)** 
MAKKKIISAILMSTVILSAAAPLSGVYAMETDTLLLWVLLLWVPGSTGDAAQPARRARRTKLAAYARKAARQARAPV
KIPNIGNVMNKFEILGVVGEGAYGVVLKCRHKETHEIVAIKKFKDSEENEEVKETTLRELKMLRTLKQENIVELKEA
FRRRGKLYLVFEYVEKNMLELLEEMPNGVPPEKVKSYIYQLIKAIHWCHKNDIVHRDIKPENLLISHNDVLKLCDFG
FARNLSEGNNANYTEYVATRWYRSPELLLGAPYGKSVDMWSVGCILGELSDGQPLFPGESEIDQLFTIQKVLGPLPS
EQMKLFYSNPRFHGLRFPAVNHPQSLERRYLGILNSVLLDLMKNLLKLDPADRYLTEQCLNHPTFQTQRLLDRSPSR
SAKRKPYHVESSTLSNRNQAGKSTALQSHHRSNSKDIQNLSVGLPRADEGLPANESFLNGNLAGASLSPLHTKTYQA
SSQPGSTSKDLTNNNIPHLLSPKEAKSKTEFDFNIDPKPSEGPGTKYLKSNSRSQQNRHSFMESSQSKAGTLQPNEK
QSRHSYIDTIPQSSRSPSYRTKAKSHGALSDSKSVSNLSEARAQIAEPSTSRYFPSSCLDLNSPTSPTPTRHSDTRT
LLSPSGRNNRNEGTLDSRRTTTRHSKTMEELKLPEHMDSSHSHSLSAPHESFSYGLGYTSPFSSQQRPHRHSMYVTR
DKVRAKGLDGSLSIGQGMAARANSLQLLSPQPGEQLPPEMTVARSSVKETSREGTSSFHTRQKSEGGVYHDPHSDDG
TAPKENRHLYNDPVPRRVGSFYRVPSPRPDNSFHENNVSTRVSSLPSESSSGTNHSKRQPAFDPWKSPENISHSEQL
KEKEKQGFFRSMKKKKKSQTVPNSDSPDLLTLQKSIHSASTPSSRPKEWRPEKISDLQTQSQPLKSLRKLLHLSSA
SNHPASSDPRFQPLTAQQTKNSFSEIRIHPLSQASGGSSNIRQEPAPKGRPALQLPGQMDPGWHVSSVTRSATEGPS
YSEQLGAKSGPNGHPYNRTNRSRMPNLNDLKETALSRGSRADYKDHDGDYKDHDIDYKDDDDK-
 TATk (SEQ ID No. 26)
YARKAARQARA
Signal peptide of USP45 (SEQ ID No. 27)
MAKKKIISAILMSTVILSAAAPLSGVYA
**Usp45 protein (Lactococcus cremoris subsp. cremoris NZ9000; SEQ ID. No 28)** 
MKKKIISAILMSTVILSAAAPLSGVYADTNSDIAKQDATISSAQSAKAQAQAQVDSLQSKVDSLQQKQTSTKAQIAK
IESEAKALNAQIATLNESIKERTKTLEAQARSAQVNSSATNYMDAVVNSKSLTDVIQKVTAIATVSSANKQMLEQQE
KEQKELSQKSETVKKNYNQFVSLSQSLDSQAQELTSQQAELKVATLNYQATIATAQDKKQALLDEKAAAEKAAQEAA
KKQAAYEAQQKEAAQAQAASTAATAKAVEAATSSASASSSQAPQVSTSTDNTTSNASASNSSNSSSNSSSSSSSSS
SSSSSSNSNAGGNTNSGTSTGNTGGTTTGGSGINSSPIGNPYAGGGCTDYVWQYFAAQGIYIRNIMPGNGGQWASNG
PAQGVLHVVGAAPGVIASSFSADFVGYANSPYGHVAIVKSVNSDGTITIKEGGYGTTWWGHERTVSASGVTFLMPN
 **CDKL5 protein (SEQ ID No 29)** 
KIPNIGNVMNKFEILGVVGEGAYGVVLKCRHKETHEIVAIKKFKDSEENEEVKETTLRELKMLRTLKQENIVELKEA
FRRRGKLYLVFEYVEKNMLELLEEMPNGVPPEKVKSYIYQLIKAIHWCHKNDIVHRDIKPENLLISHNDVLKLCDFG
FARNLSEGNNANYTEYVATRWYRSPELLLGAPYGKSVDMWSVGCILGELSDGQPLFPGESEIDQLFTIQKVLGPLPS
EQMKLFYSNPRFHGLRFPAVNHPQSLERRYLGILNSVLLDLMKNLLKLDPADRYLTEQCLNHPTFQTQRLLDRSPSR
SAKRKPYHVESSTLSNRNQAGKSTALQSHHRSNSKDIQNLSVGLPRADEGLPANESFLNGNLAGASLSPLHTKTYQA
SSQPGSTSKDLTNNNIPHLLSPKEAKSKTEFDFNIDPKPSEGPGTKYLKSNSRSQQNRHSFMESSQSKAGTLQPNEK
QSRHSYIDTIPQSSRSPSYRTKAKSHGALSDSKSVSNLSEARAQIAEPSTSRYFPSSCLDLNSPTSPTPTRHSDTRT
LLSPSGRNNRNEGTLDSRRTTTRHSKTMEELKLPEHMDSSHSHSLSAPHESFSYGLGYTSPFSSQQRPHRHSMYVTR
DKVRAKGLDGSLSIGQGMAARANSLQLLSPQPGEQLPPEMTVARSSVKETSREGTSSFHTRQKSEGGVYHDPHSDDG
TAPKENRHLYNDPVPRRVGSFYRVPSPRPDNSFHENNVSTRVSSLPSESSSGTNHSKRQPAFDPWKSPENISHSEQL
KEKEKQGFFRSMKKKKKKSQTVPNSDSPDLLTLQKSIHSASTPSSRPKEWRPEKISDLQTQSQPLKSLRKLLHLSSA
SNHPASSDPRFQPLTAQQTKNSFSEIRIHPLSQASGGSSNIRQEPAPKGRPALQLPDGGCDGRRQRHHSGPQDRRFM
LRTTEQQGEYFCCGDPKKPHTPCVPNRALHRPISSPAPYPVLQVRGTSMCPTLQVRGTDAFSCPTQQSGFSFFVRHV
MREALIHRAQVNQAALLTYHENAALTGK
 **CDKL5 polypeptide (SEQ ID No 30)**

KIPNIGNVMNKFEILGVVGEGAYGVVLKCRHKETHEIVAIKKFKDSEENEEVKETTLRELKMLRTLKQENIVELKEA
FRRRGKLYLVFEYVEKNMLELLEEMPNGVPPEKVKSYIYQLIKAIHWCHKNDIVHRDIKPENLLISHNDVLKLCDFG
FARNLSEGNNANYTEYVATRWYRSPELLLGAPYGKSVDMWSVGCILGELSDGQPLFPGESEIDQLFTIQKVLGPLPS
EQMKLFYSNPRFHGLRFPAVNHPQSLERRYLGILNSVLLDLMKNLLKLDPADRYLTEQCLNHPTFQTQRLLDRSPSR
SAKRKPYHVESSTLSNRNQAGKSTALQSHHRSNSKDIQNLSVGLPRADEGLPANESFLNGNLAGASLSPLHTKTYQA
SSQPGSTSKDLTNNNIPHLLSPKEAKSKTEFDFNIDPKPSEGPGTKYLKSNSRSQQNRHSFMESSQSKAGTLQPNEK
QSRHSYIDTIPQSSRSPSYRTKAKSHGALSDSKSVSNLSEARAQIAEPSTSRYFPSSCLDLNSPTSPTPTRHSDTRT
LLSPSGRNNRNEGTLDSRRTTTRHSKTMEELKLPEHMDSSHSHSLSAPHESFSYGLGYTSPFSSQQRPHRHSMYVTR
DKVRAKGLDGSLSIGQGMAARANSLQLLSPQPGEQLPPEMTVARSSVKETSREGTSSFHTRQKSEGGVYHDPHSDDG
TAPKENRHLYNDPVPRRVGSFYRVPSPRPDNSFHENNVSTRVSSLPSESSSGTNHSKRQPAFDPWKSPENISHSEQL
KEKEKQGFFRSMKKKKKSQTVPNSDSPDLLTLQKSIHSASTPSSRPKEWRPEKISDLQTQSQPLKSLRKLLHLSSA
SNHPASSDPRFQPLTAQQTKNSFSEIRIHPLSQASGGSSNIRQEPAPKGRPALQLPGQMDPGWHVSSVTRSATEGPS
YSEQLGAKSGPNGHPYNRTNRSRMPNLNDLKETAL

### REFERENCES

Bachmanov, A. A., et al., 2002. Food intake, water intake, and drinking spout side preference of 28 mouse strains. Behav Genet. 32, 435-43.
Berlec, A., et al., 2017. Dextran sulphate sodium colitis in C57BL/6J mice is alleviated by Lactococcus lactis and worsened by the neutralization of Tumor necrosis Factor alpha. Int Immunopharmacol. 43, 219-226.
Bertani, I., et al., 2006. Functional consequences of mutations in CDKL5, an X-linked gene involved in infantile spasms and mental retardation. J Biol Chem. 281, 32048-56.
Bolhassani, A., et al., 2017. In vitro and in vivo delivery of therapeutic proteins using cell penetrating peptides. Peptides. 87, 50-63.
Bron, P. A., Kleerebezem, M., 2018. Lactic Acid Bacteria for Delivery of Endogenous or Engineered Therapeutic Molecules. Front Microbiol. 9, 1821.
Calvanese, M., et al., 2022. Soluble Recombinant Protein Production in Pseudoalteromonas haloplanktis TAC125: The Case Study of the Full-Length Human CDKL5 Protein. Methods Mol Biol. 2406, 219-232.
Cano-Garrido, O., et al., 2015. Lactic acid bacteria: reviewing the potential of a promising delivery live vector for biomedical purposes. Microb Cell Fact. 14, 137.
Charbonneau, M. R., et al., 2020. Developing a new class of engineered live bacterial therapeutics to treat human diseases. Nat Commun. 11, 1738.
de Ruyter, P. G., et al., 1996. Controlled gene expression systems for Lactococcus lactis with the food-grade inducer nisin. Appl Environ Microbiol. 62, 3662-7.
Engler, C., et al., 2008. A one pot, one step, precision cloning method with high throughput capability. PLoS One. 3, e3647.
Fehr, S., et al., 2013. The CDKL5 disorder is an independent clinical entity associated with early-onset encephalopathy. Eur J Hum Genet. 21, 266-73.
Guerrini, R., Parrini, E., 2012. Epilepsy in Rett syndrome, and CDKL5- and FOXG1-gene-related encephalopathies. Epilepsia. 53, 2067-78.
Holo, H., Nes, I. F., 1995. Transformation of Lactococcus by electroporation. Methods Mol Biol. 47, 195-9.
Katayama, S., et al., 2015. Critical Determinants of Substrate Recognition by Cyclin-Dependent Kinase-like 5 (CDKL5). Biochemistry. 54, 2975-87.
Lee, C. E., et al., 2020. Rare Genetic Diseases: Nature's Experiments on Human Development. iScience. 23, 101123.
Mari, F., et al., 2005. CDKL5 belongs to the same molecular pathway of MeCP2 and it is responsible for the early-onset seizure variant of Rett syndrome. Hum Mol Genet. 14, 1935-46.
Mierau, I., Kleerebezem, M., 2005. 10 years of the nisin-controlled gene expression system (NICE) in Lactococcus lactis. Appl Microbiol Biotechnol. 68, 705-17.
Nagahara, H., et al., 1998. Transduction of full-length TAT fusion proteins into mammalian cells: TAT-p27Kip1 induces cell migration. Nat Med. 4, 1449-52.
Nguengang Wakap, S., et al., 2020. Estimating cumulative point prevalence of rare diseases: analysis of the Orphanet database. Eur J Hum Genet. 28, 165-173.
Plavec, T. V., Berlec, A., 2019. Engineering of lactic acid bacteria for delivery of therapeutic proteins and peptides. Appl Microbiol Biotechnol. 103, 2053-2066.
Rapoport, M., Lorberboum-Galski, H., 2009. TAT-based drug delivery system--new directions in protein delivery for new hopes? Expert Opin Drug Deliv. 6, 453-63.
Schwarze, S. R., et al., 1999. In vivo protein transduction: delivery of a biologically active protein into the mouse. Science. 285, 1569-72.
Skrlec, K., et al., 2017. Evasin-displaying lactic acid bacteria bind different chemokines and neutralize CXCL8 production in Caco-2 cells. Microb Biotechnol. 10, 1732-1743.
Song, A. A., et al., 2017. A review on Lactococcus lactis: from food to factory. Microb Cell Fact. 16, 55.
Szatraj, K., et al., 2017. Lactic acid bacteria - promising vaccine vectors: possibilities, limitations, doubts. J Appl Microbiol. 123, 325-339.
Trazzi, S., et al., 2018. CDKL5 protein substitution therapy rescues neurological phenotypes of a mouse model of CDKL5 disorder. Hum Mol Genet. 27, 1572-1592.
van Asseldonk, M., et al., 1993. Functional analysis of the Lactococcus lactis usp45 secretion signal in the secretion of a homologous proteinase and a heterologous alpha-amylase. Mol Gen Genet. 240, 428-34.
Vesa, T., et al., 2000. Pharmacokinetics of Lactobacillus plantarum NCIMB 8826, Lactobacillus fermentum KLD, and Lactococcus lactis MG 1363 in the human gastrointestinal tract. Aliment Pharmacol Ther. 14, 823-8.
Wang, M., et al., 2016. Lactic acid bacteria as mucosal delivery vehicles: a realistic therapeutic option. Appl Microbiol Biotechnol. 100, 5691-701.
Xia, H., et al., 2001. The HIV Tat protein transduction domain improves the biodistribution of beta-glucuronidase expressed from recombinant viral vectors. Nat Biotechnol. 19, 640-4.
Zahirovic, A., et al., 2022. Dual Functionalized Lactococcus lactis Shows Tumor Antigen Targeting and Cytokine Binding in Vitro. Front Bioeng Biotechnol. 10, 822823.

## Claims

1. A non-pathogenic bacterium genetically modified to express a Protein Fusion Domain (PTD)-fusion protein wherein said non-pathogenic bacterium comprises a gene construct coding for a secretion signal peptide and for a PTD-fusion protein, said gene construct being under the control of a suitable promoter.

2. The non-pathogenic bacterium according to claim 1 wherein the encoded PTD-fusion protein is a TATk-fusion protein.

3. The non-pathogenic bacterium according to claim 2 wherein the encoded TATk-fusion protein comprises:
- a secretion signal peptide of USP45 with at least 70%, at least 80% or at least 90% sequence identity to SEQ ID No. 27;
- a TATk polypeptide sequence with at least 70%, at least 80% or at least 90% sequence identity to SEQ ID No. 26;
- a protein suitable for protein replacement therapy, preferably said protein being selected from a CDKL5 polypeptide sequence, alpha1-Antitrypsin, β-Glucocerebrosidase, Adenosine Deaminase, Alpha-Galactosidase A, Acid alpha-Glucosidase, α-L-Iduronidase, Iduronate-2-Sulfatase, N-Acetylgalactosamine-6 Sulfatase, N-Acetylgalactosamine-4 Sulfatase, Lysosomal Acid Lipase, MECP2 (methyl-CpG binding protein 2), KDM5C (lysine demethylase 5C), CASK (Calcium/calmodulin-dependent serine protein kinase), SERPINA1 (serine protease inhibitor a1-antitrypsin (AAT) protein), DNAI1 (Dynein Axonemal Intermediate Chain 1), CYBB (cytochrome b-245, beta chain), XPA (DNA repair protein complementing XP-A cells);
wherein the TATk is operatively coupled to the protein suitable for protein replacement therapy.

4. The non-pathogenic bacterium according to any one of previous claims wherein the encoded PTD-fusion protein further comprises a reporter protein polypeptide wherein the reporter protein polypeptide is operatively coupled to the N-terminus or to the C-terminus of the PTD-fusion protein.

5. The non-pathogenic bacterium according to any one of claims 2 to 4 wherein the encoded TATk fusion protein has or comprises a polypeptide sequence with at least 70%, at least 80% or at least 90% sequence identity to SEQ ID No. 25.

6. The non-pathogenic bacterium according to any one of previous claims being a lactic acid bacterium (LAB), preferably said LAB is *Lactococcus lactis.*

7. The non-pathogenic bacterium according to any one of claims 1 to 6 for use as a medicament.

8. The non-pathogenic bacterium according to claim 7 for use as a delivery vector for protein replacement therapy in the treatment of genetic disease, preferably in the treatment of rare monogenic diseases.

9. The non-pathogenic bacterium for use according to claim 8 wherein the rare monogenic diseases are neurological diseases preferably selected from Fragile-X syndrome, CDKL5 deficiency disorder, Rett syndrome, Lysosomal storage diseases, Claes-Jensen type (MRXSCJ); CASK-related disorders; α1-antitrypsin deficiency; Kartagener syndrome; Chronic Granulomatous Disease; Xeroderma Pigmentosum, Gaucher disease, Pompe disease, ADA deficiency.

10. A pharmaceutical composition comprising a therapeutically effective amount of the non-pathogenic bacterium according to any one of claims 1 to 6 and a pharmaceutically acceptable carrier.

11. The pharmaceutical composition according to claim 10 for use according to any one of claims 7 to 9.

12. A method for producing the non-pathogenic bacterium according to any one of claims 2 to 6 comprising introducing into said lactic acid bacterium a gene construct coding for a secretion signal peptide and for a TATk-fusion protein, said gene construct being under the control of a suitable promoter, preferably wherein the genetic construct comprises a sequence with at least 70%, at least 80% or at least 90% sequence identity to any one of SEQ ID No 16, SEQ ID No. 18, SEQ ID NO 24.

13. The method according to claim 12 wherein said gene construct is in a plasmid vector capable of replicating in said lactic acid bacterium or said gene construct is integrated into the genome of said lactic acid bacterium.

14. A food product comprising the non-pathogenic bacterium according to any one of claims 1 to 6, preferably said food product being selected from the group consisting of milk, yogurt, curd, cheese, fermented milks, milk based fermented products and milk-based powders.

15. Use of the non-pathogenic bacterium according to any one of claims 1 to 6 as base ingredient for the preparation of nutraceutical products.
